# EUROPEAN PATENT APPLICATION

(11) **EP 4 487 894 A1**
(43) Date of publication of application: **08.01.2025**
(21) Application number: 23760120.8
(22) Date of filing: 24.02.2023
(51) Int. Cl.: A61M 25/01, A61F 2/966

(54) **CATHETER AND DELIVERY SYSTEM**

(30) Priority: 28.02.2022 JP 2022030220; 25.03.2022 JP 2022050277
(71) Applicant: SB-Kawasumi Laboratories, Inc., Kawasaki-shi, Kanagawa 210-8602 (JP); Sumitomo Bakelite Co.Ltd., Shinagawa-ku Tokyo 140-0002 (JP)
(72) Inventor: MIYAHISA, Masako, Kawasaki-shi, Kanagawa 210-8602 (JP); YOKOTA, Tomoaki, Kawasaki-shi, Kanagawa 210-8602 (JP); MUKAI, Tomokazu, Kawasaki-shi, Kanagawa 210-8602 (JP)
(74) Representative: Müller-Boré & Partner Patentanwälte PartG mbB
(86) International application number: PCT/JP2023/006825
(87) International publication number: WO 2023/163122

(57) **Abstract**

Provided are a catheter and a delivery system that can accurately place a tubular indwelling device at an indwelling target site in a body lumen. A catheter 1 for delivering a colonic stent 2 includes: a sheath 11; an elongated inner tube 12 that is configured to move inside the sheath in an axial direction; a plurality of guide ribs 31 to 33 that are disposed on the inner tube to be spaced apart from each other in the axial direction; and a linear member (a restraining strap 21 and a holding wire 22) that maintains axially at least a portion of the colonic stent in a contracted state, with the colonic stent released from the sheath. The plurality of guide ribs each include a tube attachment portion 40 that is eccentrically provided with respect to a circumscribed circle of the guide rib in a cross-section orthogonal to the axial direction and to which the inner tube is attached, and a guide portion 50 through which the linear member is inserted, and are disposed to be displaced from each other in a circumferential direction with respect to the inner tube.

## Description

### Technical Field

The present invention relates to a catheter and a delivery system.

### Background Art

Conventionally, a tubular indwelling device (generally called a "stent") has been known to be placed in a stenotic part or an occluded part occurring in a digestive tract, such as an esophagus, a stomach, a small intestine, a colon, and a bile duct, or a blood vessel (hereinafter, referred to as a "body lumen") to dilate a lesion site and maintain patency of the body lumen (for example, refer to Patent Document 1). The tubular indwelling device is delivered to an indwelling target site using a catheter.

### Prior Art Document

### Patent Document

[Patent Document 1] PCT Japanese Translation Patent Publication No. 2005-514106

### Summary of Invention

### Technical Problem

Meanwhile, the tubular indwelling device is attached to an axial member of the catheter in a radially contracted state and housed in a sheath, but when releasing the tubular indwelling device from the sheath in a state in which a portion of the tubular indwelling device in an axial direction is contracted, there is a concern that an outer surface of the tubular indwelling device may come into contact with an inner surface of the sheath, causing the tubular indwelling device to deviate in the axial direction with respect to the axial member. In particular, when releasing the tubular indwelling device from the sheath to a curved indwelling target site, a frictional force between the sheath and the tubular indwelling device increases on an outer-side surface side having a larger curvature radius, and the tubular indwelling device is more likely to deviate from the axial member. As a result, it is difficult to accurately place the tubular indwelling device at the indwelling target site in the body lumen.

An object of the present invention is to provide a catheter and a delivery system that can accurately place a tubular indwelling device at an indwelling target site in a body lumen.

### Solution to Problem

According to the present invention, there is provided a catheter for delivering a tubular indwelling device to be placed in a body lumen, the catheter including:
a sheath;
an elongated axial member that is configured to move inside the sheath in an axial direction;
a plurality of guide ribs that are disposed on the axial member to be spaced apart from each other in the axial direction and are configured to position the tubular indwelling device, which is in a radially contracted state inside the sheath, in the axial direction of the axial member; and
a linear member that maintains axially at least a portion of the tubular indwelling device in a contracted state, with the tubular indwelling device released from the sheath,
in which the plurality of guide ribs each include
   an attachment portion that is eccentrically provided with respect to a circumscribed circle of the guide rib in a cross-section orthogonal to the axial direction and to which the axial member is attached, and
   a guide portion through which the linear member is inserted, and
the guide ribs are disposed to be displaced from each other in a circumferential direction with respect to the axial member.

According to the present invention, there is provided a delivery system including:
a tubular indwelling device to be placed in a body lumen; and
a catheter for delivering the tubular indwelling device into the body lumen,
in which the catheter includes
   a sheath,
   an elongated axial member that is configured to move inside the sheath in an axial direction,
   a plurality of guide ribs that are disposed on the axial member to be spaced apart from each other in the axial direction and are configured to position the tubular indwelling device, which is in a radially contracted state inside the sheath, in the axial direction of the axial member, and
   a linear member that maintains axially at least a portion of the tubular indwelling device in a contracted state, with the tubular indwelling device released from the sheath,
the plurality of guide ribs each include
   an attachment portion that is eccentrically provided with respect to a circumscribed circle of the guide rib in a cross-section orthogonal to the axial direction and to which the axial member is attached, and
   a guide portion through which the linear member is inserted, and
the guide ribs are disposed to be displaced from each other in a circumferential direction with respect to the axial member.

According to the present invention, there is provided a catheter for delivering a tubular indwelling device to be placed in a body lumen, the catheter including:
a sheath;
an elongated axial member that is configured to move inside the sheath in an axial direction and includes a first guide portion through which a guide wire is inserted; and
a linear member that maintains axially at least a portion of the tubular indwelling device in a contracted state, with the tubular indwelling device released from the sheath,
in which the axial member includes
   a positioning portion that is provided on a portion of the axial member in the axial direction and that is disposed in a state in which the tubular indwelling device, which is in a radially contracted state inside the sheath, is positioned in the axial direction, and
   a second guide portion through which the linear member is inserted.

According to the present invention, there is provided a delivery system including:
a tubular indwelling device to be placed in a body lumen; and
a catheter for delivering the tubular indwelling device,
in which the catheter includes
   a sheath,
   an elongated axial member that is configured to move inside the sheath in an axial direction and includes a first guide portion through which a guide wire is inserted, and
   a linear member that maintains axially at least a portion of the tubular indwelling device in a contracted state, with the tubular indwelling device released from the sheath, and
the axial member includes
   a positioning portion that is provided on a portion of the axial member in the axial direction and that is disposed in a state in which the tubular indwelling device, which is in a radially contracted state inside the sheath, is positioned in the axial direction, and
   a second guide portion through which the linear member is inserted.

### Advantageous Effects of Invention

According to the present invention, the tubular indwelling device can be accurately placed at an indwelling target site of a body lumen.

### Brief Description of Drawings

Figs. 1A and 1B are views showing a configuration of a delivery system.
Fig. 2 is a schematic view showing an example of conversion means.
Fig. 3 is a schematic view showing an example of a guide rib.
Figs. 4A and 4B are schematic views showing a portion to which a colonic stent is attached in the delivery system.
Figs. 5A and 5B are perspective views showing an example of a specific disposition aspect of first to third guide ribs.
Figs. 6A to 6C are views showing circumferential displacements of the first to third guide ribs with respect to the inner tube.
Figs. 7A to 7C are schematic views showing an example of a state change during placement of the colonic stent.
Figs. 8A and 8B are views showing another example of the guide rib.
Figs. 9A and 9B are views showing a configuration of a delivery system.
Fig. 10 is a schematic view showing an example of conversion means.
Figs. 11A and 11B are schematic views showing a structure of an inner tube.
Figs. 12A and 12B are schematic views showing an example of a protruding rib disposed on the inner tube.
Figs. 13A and 13B are schematic views showing attachment aspects of the colonic stent and the conversion means with respect to the inner tube.
Figs. 14A to 14C are schematic views showing an example of a state change during placement of the colonic stent.
Figs. 15A and 15B are schematic views showing a structure of an inner tube according to Modification Example 1.
Figs. 16A and 16B are schematic views showing a structure of an inner tube according to Modification Example 2.
Fig. 17A is a schematic view showing a state in which the colonic stent is restrained by a restraining strap, and Fig. 17B is a schematic view showing a state in which restraint of the colonic stent by the restraining strap is partially released.
Figs. 18A and 18B are views illustrating restraint of an end part of the colonic stent.
Figs. 19A and 19B are views illustrating restraint of the end part of the colonic stent.
Figs. 20A to 20C are views illustrating restraint of a central part of the colonic stent.

### Description of Embodiments

Hereinafter, embodiments of the present invention will be described in detail with reference to the accompanying drawings.

### [First Embodiment]

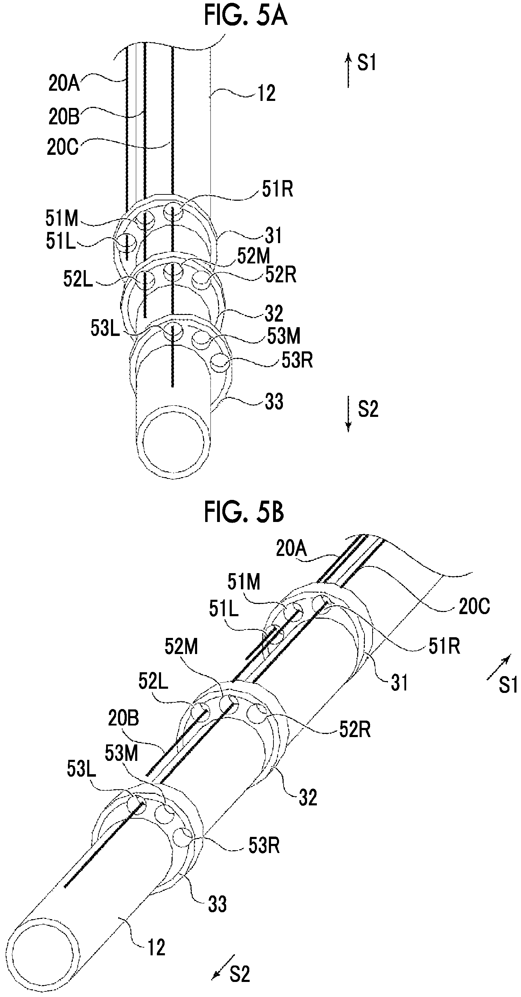

In a first embodiment, as an example of the present invention, a catheter 1 and a delivery system 100 for placing a colonic stent 2, which is a tubular indwelling device, in a colon C in order to treat occlusion (stenosis) by pushing and expanding a lesion site L of the colon C (for example, an occluded part or a stenotic part of the colon, refer to Fig. 5A and the like) radially outward will be described.

A delivery device for placing the colonic stent 2 at an indwelling target site is referred to as a "catheter 1", and a system in which the colonic stent 2 is loaded into the catheter 1 is referred to as a "delivery system 100".

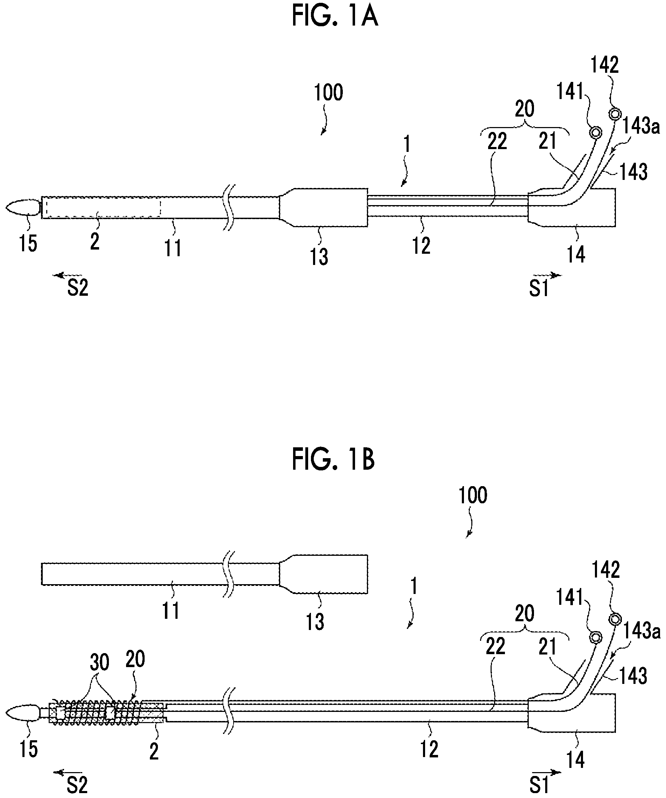

Figs. 1A and 1B are views showing a configuration of the delivery system 100. Fig. 1A shows a state in which the delivery system 100 is assembled. Fig. 1B shows a state in which the delivery system 100 is disassembled. In Figs. 1A and 1B, in order to facilitate the understanding of the invention, a size (a length, a diameter dimension, and the like), a shape, or the like of each member constituting the delivery system 100 are schematically shown. In the following description, descriptions will be provided by representing a right side in Figs. 1A and 1B as a proximal side S1 and a left side as a distal side S2.

As shown in Figs. 1A and 1B, the delivery system 100 includes the catheter 1 and the colonic stent 2. The delivery system 100 is used, for example, endoscopically by being inserted into a forceps hole of an endoscope when placing the colonic stent 2 in the colon.

The colonic stent 2 has a tubular shape to define a tubular flow path through which a digestive substance flows. The colonic stent 2 is, for example, a bare stent that is formed of only a skeleton. The skeleton is a reinforcing member for maintaining an expanded state of the colonic stent 2 and is formed to be self-expandable from a contracted state in which the skeleton is contracted inward to an expanded state in which the skeleton is expanded outward, in a radial direction substantially orthogonal to an axial direction.

The skeleton is formed, for example, by weaving a wire material into a tubular shape. The skeleton is formed by, for example, weaving two wire materials that helically extend while being folded back in a zigzag shape (Z-like shape) at a predetermined pitch into a diamond-like mesh shape (fence-like shape) such that bending portions (a convex peak at on one end side in the axial direction and a convex valley on the other end side in the axial direction) mesh with each other. In this case, in a state in which tension is applied to the colonic stent 2 in the axial direction, the bending portions of the wire materials forming the mesh closely intersect each other, so that elongation in the axial direction is restricted.

The skeleton is not limited to the above-described skeleton structure and may be configured as, for example, a helical skeleton in which a single wire material is helically wound while being bent in a zigzag shape (Z-like shape) such that a peak and a valley are alternately formed. In addition, the skeleton may be formed by performing laser processing on a metal cylindrical member. Further, the wire material forming the skeleton may be a plurality of wire materials.

Examples of the material forming the skeleton include known metals or metal alloys represented by stainless steel, a nickel-titanium alloy (nitinol), a titanium alloy, and the like. In addition, an alloy material having X-ray contrast properties may be used. In this case, the position of the colonic stent 2 can be confirmed from an outside of a body. The skeleton may be formed of a material other than the metal material (for example, ceramics, resins, or the like).

The material, the wire type (for example, a round wire material such as wire, or angular wire material by laser cutting), the cross-sectional area (corresponding to the wire diameter in a case of the round wire material), and the number of bends and the bending shape in the circumferential direction (the number and shape of bending portions) of the wire material forming the skeleton, an interval between wire materials in the axial direction (skeleton amount per unit length), and the like are selected as appropriate, for example, based on criteria such as storage properties in the sheath required for the colonic stent 2 according to the indwelling site, release properties from the sheath, indwelling capability (corresponding to an expansion force), and flexibility (ease of bending).

The catheter 1 includes a sheath 11, an inner tube 12, a first operation part 13, a second operation part 14, a distal end tip 15, conversion means 20, a guide rib 30, and the like.

The sheath 11 is, for example, a tubular member formed of a material having flexibility. The inner tube 12 is, for example, an elongated axial member formed of a material having moderate hardness and flexibility, such as a resin or a metal. A proximal end of the sheath 11 is connected to the first operation part 13, and a proximal end of the inner tube 12 is connected to the second operation part 14. In addition, the distal end tip 15 is disposed at a distal end of the inner tube 12.

The inner tube 12 is inserted into the sheath 11 and is disposed to extend to the distal side S2 of the sheath 11. The sheath 11 and the inner tube 12 are configured to move relative to each other in the axial direction of the sheath 11 through the operations of the first operation part 13 and the second operation part 14.

Although not shown, for example, a lumen for passing a guide wire, a lumen for passing the conversion means 20 for expanding the colonic stent 2 in a contracted state at an affected part, and the like may be formed in the inner tube 12 and the distal end tip 15 along the axial direction.

The colonic stent 2 is attached to an end part of the inner tube 12 on the distal side S2 and is housed in the sheath 11 in a radially expandable contracted state. Specifically, the colonic stent 2 is put into a contracted state by being radially folded while elongating in the axial direction, and is housed in the sheath 11. At this time, the colonic stent 2 is held in a contracted state by the conversion means 20.

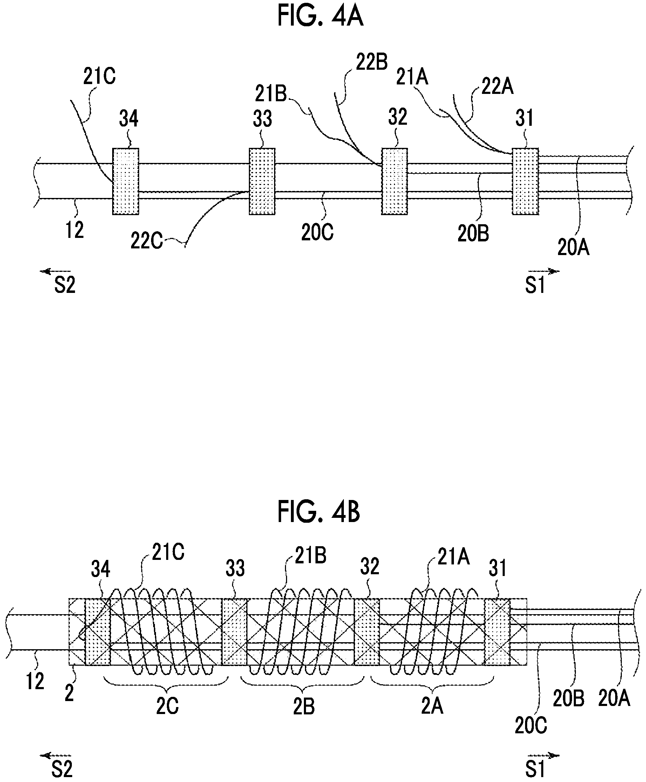

The conversion means 20 is configured to maintain axially at least a portion (for example, a third stent portion 2C or the like shown in Fig. 4B) of the colonic stent 2 in a contracted state, with the colonic stent 2 released from the sheath 11, and to convert the portion from a contracted state to an expanded state.

The guide rib 30 is disposed on an outer peripheral surface of a stent attachment portion, which is the end part of the inner tube 12 on the distal side S2. In the present embodiment, a plurality of the guide ribs 30 are disposed to be spaced apart from each other in the axial direction with respect to the inner tube 12. The guide rib 30 is fixed to the inner tube 12 by, for example, adhesion, welding, or the like.

The guide rib 30 positions the colonic stent 2, which is in a contracted state, in the axial direction of the inner tube 12 and holds the conversion means 20. The conversion means 20 extends along the inner tube 12 by being inserted through and held by the plurality of guide ribs 30.

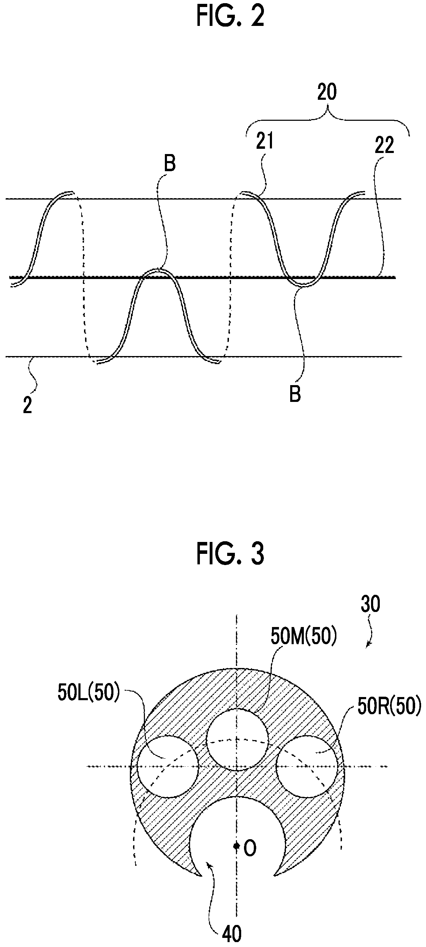

In addition, the guide rib 30 is configured such that, for example, in a state in which the colonic stent 2 is attached to the inner tube 12, the skeleton of the colonic stent 2 is caught on an end surface of the guide rib 30. Consequently, the colonic stent 2 can be positioned in the axial direction of the inner tube 12, and the colonic stent 2 is movable in conjunction with the inner tube 12. Further, when releasing the colonic stent 2 from the sheath 11 and when pulling out a holding wire 22 (refer to Fig. 2) which is the conversion means 20, the skeleton of the colonic stent 2 in a contracted state is caught on the end surface of the guide rib 30. Therefore, the colonic stent 2 can be expanded without deviation of the colonic stent 2 in the axial direction with respect to the inner tube 12.

Fig. 2 is a schematic view showing an example of the conversion means 20. As shown in Fig. 2, the conversion means 20 is composed of a restraining strap 21 wound around an outer peripheral surface of the colonic stent 2 and the holding wire 22 engaged with the restraining strap 21. The conversion means 20 is attached to, for example, the colonic stent 2 mounted on the inner tube 12 such that the colonic stent 2 is radially contracted.

The restraining strap 21 and the holding wire 22 are formed of, for example, a material having a predetermined strength and rigidity. As the restraining strap 21 and the holding wire 22, for example, a sewing thread such as a nylon fiber or a fluorine fiber, a metal wire made of a nickel-titanium alloy or stainless steel, or a strap-like member made of a resin can be applied. It is preferable that the restraining strap 21 and the holding wire 22 are formed of different materials in order to improve slipperiness when being pulled out.

The colonic stent 2 is held in a contracted state by a portion of the restraining strap 21 and the holding wire 22 on the distal side S2. End parts of the restraining strap 21 and the holding wire 22 on the proximal side S1 are each extracted from, for example, a branch port 143a on one side of a Y connector 143 of the second operation part 14 and are connected to pulling-out operation portions 141 and 142. The pulling-out operation portions 141 and 142 are pulled in a predetermined direction (for example, in an upper right direction in Fig. 1A, or the like) so that the restraining strap 21 and the holding wire 22 can be pulled out. A dial (not shown) may be provided on the second operation part 14, and the holding wire 22 may be pulled out while making fine adjustments through a rotational operation of the dial, or the like.

In the example shown in Fig. 2, the restraining strap 21 is wound around an outer peripheral surface of a portion (for example, the third stent portion 2C or the like shown in Fig. 4B) of the colonic stent 2 such that the restraining strap 21 bends and reverses a winding direction thereof for each turn, for example. Meanwhile, the holding wire 22 is disposed along the axial direction of the colonic stent 2 and is engaged with a bending portion B formed at the restraining strap 21. That is, the restraining strap 21 is wound in an aspect in which the restraining strap 21 cannot hold a wound state by itself, and is held in a non-detachable manner by being engaged with the holding wire 22. Therefore, in a case where the engagement between the restraining strap 21 and the holding wire 22 is released, the restraining strap 21 is naturally detached from the colonic stent 2. As a result, a portion of the colonic stent 2 is released from the contracted state and transitions to the expanded state.

Fig. 3 is a schematic view showing an example of the guide rib 30. The guide rib 30 is configured as a single molded member and has, for example, a disc shape. The guide rib 30 is formed, for example, by injection molding in which a molten resin is injected into a mold and cooled and solidified. In addition, for example, the guide rib 30 may be formed by extrusion or cutting of a metal component, or by three-dimensional shaping. Further, a resin raw material may contain a material that enhances contrast properties. A maximum outer diameter of the guide rib 30, that is, an outer diameter of a circle circumscribing the guide rib 30 is set to be smaller than an inner diameter of the sheath 11 even in a state in which the colonic stent 2 is mounted on a peripheral surface of the guide rib 30.

The guide rib 30 includes a tube attachment portion 40 and a guide portion 50. The guide rib 30 is configured as a single molded member, and the tube attachment portion 40 and the guide portion 50 are inseparable from each other, leading to excellent handleability and safety.

The tube attachment portion 40 is a portion to be attached to the inner tube 12. The tube attachment portion 40 has an arc-shaped cross-section and is formed in the outer peripheral surface of the guide rib 30 by being cut into a groove-like notch. That is, the tube attachment portion 40 is eccentrically provided with respect to the circumscribed circle of the guide rib 30.

The arc shape of the tube attachment portion 40 is a shape that conforms to an outer shape of the inner tube 12. In addition, a central angle of the arc shape is preferably, for example, 180° or more (that is, larger than a semicircular arc) in order to prevent detachment from the inner tube 12. The inner tube 12 is inserted through and fixed to the tube attachment portion 40 so that the guide rib 30 is mounted on the inner tube 12.

The guide portion 50 is a through-hole through which the conversion means 20 (the restraining strap 21 and the holding wire 22) is inserted to hold the conversion means 20. By eccentrically forming the tube attachment portion 40, a forming region of the guide portion 50 is eccentrically formed, so that the guide portion 50 can be easily formed. An eccentricity ratio, which indicates a degree of eccentricity of the tube attachment portion 40, is, for example, preferably 1.0 to 3.5 and more preferably 1.5 to 2.5. Here, the eccentricity ratio is represented by a maximum length from the tube attachment portion 40 to an outer peripheral edge of the guide rib 30 in a radial direction of the tube attachment portion 40/ (a radius of the guide rib 30 - a radius of the tube attachment portion 40) .

The number of the guide portions 50 is set, for example, according to the number of the conversion means 20 inserted through the guide rib 30. The guide rib 30 shown in Fig. 3 includes three guide portions 50, and three conversion means 20 can be individually inserted and guided. Consequently, it is possible to suppress first to third conversion means 20A to 20C from being entangled with each other, and it is possible to suppress resistance when the pulling-out. Therefore, among first to third holding wires 22A to 22C, only a desired holding wire (for example, the first holding wire 22A or the like) can be smoothly pulled out. In addition, among first to third restraining straps 21A to 21C, only a desired restraining strap (for example, the first restraining strap 21A or the like) can be smoothly pulled out. Hereinafter, in a case of distinguishing between the three guide portions 50, the three guide portions 50 are referred to as a "left guide portion 50L", a "middle guide portion 50M", and a "right guide portion 50R".

In a case where a plurality of guide portions 50 are provided, the plurality of guide portions 50 are disposed on, for example, a circle concentric with a center O (which is also substantially the same as the center of the tube attachment portion 40) of the inner tube 12 to be inserted through the tube attachment portion 40.

In addition, a relative positional relationship between two guide portions 50 adjacent to each other is the same. That is, the relative positional relationship between the left guide portion 50L and the middle guide portion 50M, and the relative position relationship between the middle guide portion 50M and the right guide portion 50R are the same. In the guide rib 30 shown in Fig. 3, the adjacent guide portions 50 have a positional relationship in which the rotation of 45° is performed therebetween about the center O. Consequently, in a case where the plurality of guide ribs 30 having the same structure are disposed to be displaced from each other in the circumferential direction with respect to the inner tube 12, the plurality of conversion means 20 can be held in parallel to each other, and the interference between the plurality of conversion means 20 can be prevented. In particular, in a case where the guide portions 50 are disposed to overlap with each other in the axial direction, the conversion means 20 is also parallel to the axial direction.

Figs. 4A and 4B are schematic views showing a portion to which the colonic stent 2 is attached in the delivery system 100. As shown in Figs. 4A and 4B, the first to third conversion means 20A to 20C are provided to correspond to first stent portion 2A to the third stent portion 2C in which the colonic stent 2 is divided into three in the axial direction. The first stent portion 2A is a portion located on the most proximal side S1, the third stent portion 2C is a portion located on the most distal side S2, and the second stent portion 2B is a portion between the first stent portion 2A and the second stent portion 2B.

The first to third conversion means 20A to 20C include the first to third restraining straps 21A to 21C and the first to third holding wires 22A to 22C, respectively. In Fig. 4B, the first to third holding wires 22A to 22C are omitted. The first to third conversion means 20A to 20C are each configured to independently operate, and the expansion of a stent portion to be expanded (for example, the first stent portion 2A or the like), among the first to third stent portions 2A to 2C of the colonic stent 2, can be flexibly controlled while confirming an indwelling state or the like.

As shown in Figs. 4A and 4B, the catheter 1 includes first to fourth guide ribs 31 to 34 as the guide ribs 30. For the first to fourth guide ribs 31 to 34, guide ribs having the same structure may be used, or guide ribs having different structures may be used in which the number of guide portions 50 is set according to the number of the conversion means 20 to be inserted. Here, a case where the first to fourth guide ribs 31 to 34 have the same structure will be described. The guide rib 30 having the structure shown in Fig. 3 is applied to the first to fourth guide ribs 31 to 34, for example. Hereinafter, in a case of distinguishing between the left guide portions 50L, the middle guide portions 50M, and the right guide portions 50R provided in the first to third guide ribs 31 to 33, the left guide portions 50L are referred to as left guide portions 51L to 53L, the middle guide portions 50M are referred to as middle guide portions 51M to 53M, and the right guide portions 50R are referred to as right guide portions 51R to 53R.

The first to fourth guide ribs 31 to 34 are disposed to be spaced apart from each other in order from the proximal side S1 along the axial direction of the inner tube 12. The first stent portion 2A of the colonic stent 2 is located between the first guide rib 31 and the second guide rib 32, the second stent portion 2B is located between the second guide rib 32 and the third guide rib 33, and the third stent portion 2C is located between the third guide rib 33 and the fourth guide rib 34.

Here, the second guide rib 32 adjacent to the first guide rib 31 is displaced clockwise by 45° along the circumferential direction with respect to the first guide rib 31, with the center O of the tube attachment portion 40 as a rotation center. In addition, the third guide rib 33 located two adjacent to the first guide rib 31 is displaced by 90° clockwise along the circumferential direction with respect to the first guide rib 31, with the center O of the tube attachment portion 40 as the rotation center.

In the first guide rib 31, with the left guide portion 51L as a reference, the middle guide portion 51M and the right guide portion 51R are located at positions respectively rotated by 45° and 90° in a clockwise direction, with the center O of the tube attachment portion 40 as the rotation center.

Therefore, it can be said that the displacement angles of the second guide rib 32 and the third guide rib 33 with respect to the first guide rib 31 are defined based on the relative positional relationship between the left guide portion 51L, the middle guide portion 51M, and the right guide portion 51R provided in the first guide rib 31. The same can be said for the displacement angles of the first guide rib 31 and the third guide rib 33 with respect to the second guide rib 32, and the displacement angles of the first guide rib 31 and the second guide rib 32 with respect to the third guide rib 33. As a result, it is possible to easily determine orientations of the first to third guide ribs 31 to 33 such that the plurality of conversion means 20 do not interfere with each other.

The first conversion means 20A is inserted only through the first guide rib 31 on the most proximal side S1. The first holding wire 22A of the first conversion means 20A is inserted through the first guide rib 31, and then is extracted to the outside of the colonic stent 2 and is disposed along the first stent portion 2A. Similar to the first holding wire 22A, the first restraining strap 21A is inserted through the first guide rib 31, and then is extracted to the outside of the colonic stent 2 and is wound around the outer peripheral surface of the first stent portion 2A from the proximal side S1 toward the distal side S2.

The second conversion means 20B is inserted through the first guide rib 31 and the second guide rib 32 in this order from the proximal side S1. The second holding wire 22B of the second conversion means 20B is inserted through the second guide rib 32, and then is extracted to the outside of the colonic stent 2 and is disposed along the second stent portion 2B. Similar to the second holding wire 22B, the second restraining strap 21B is inserted through the second guide rib 32, and then is extracted to the outside of the colonic stent 2 and is wound around the outer peripheral surface of the second stent portion 2B from the proximal side S1 toward the distal side S2.

The third conversion means 20C is inserted through the first guide rib 31, the second guide rib 32, and the third guide rib 33 in this order from the proximal side S1. The third holding wire 22C of the third conversion means 20C is inserted through the third guide rib 33, and then is extracted to the outside of the colonic stent 2 and is disposed along the third stent portion 2C. The third restraining strap 21C is further inserted through the fourth guide rib 34 and is folded back, and then is wound around the outer peripheral surface of the third stent portion 2C from the distal side S2 toward the proximal side S1.

The third restraining strap 21C does not necessarily need to be inserted through the fourth guide rib 34, and the fourth guide rib 34 may have only a function of being engaged with the colonic stent 2. In addition, the first conversion means 20A does not necessarily need to be inserted through the first guide rib 31. That is, the second and third conversion means 20B and 20C may be configured to be inserted through the first guide rib 31, and the first guide rib 31 may have only a function of suppressing bending of the inner tube 12 or the catheter 1.

In addition, it is preferable that the end surface of the fourth guide rib 34 on which the third restraining strap 21C is folded back is chamfered. Consequently, when retrieving the third restraining strap 21C folded back to the proximal side S1, kinks or damage occurring on the third restraining strap 21C due to friction with the fourth guide rib 34 can be reduced. Further, the fourth guide rib 34 may have a structure capable of storing the excess third restraining strap 21C. In addition, the fourth guide rib 34 does not necessarily need to be disposed, and the third restraining strap 21C may be inserted through the third guide rib 33, and then may be extracted to the outside of the colonic stent 2 and may be wound around the outer peripheral surface of the third stent portion 2C from the proximal side S1 toward the distal side S2.

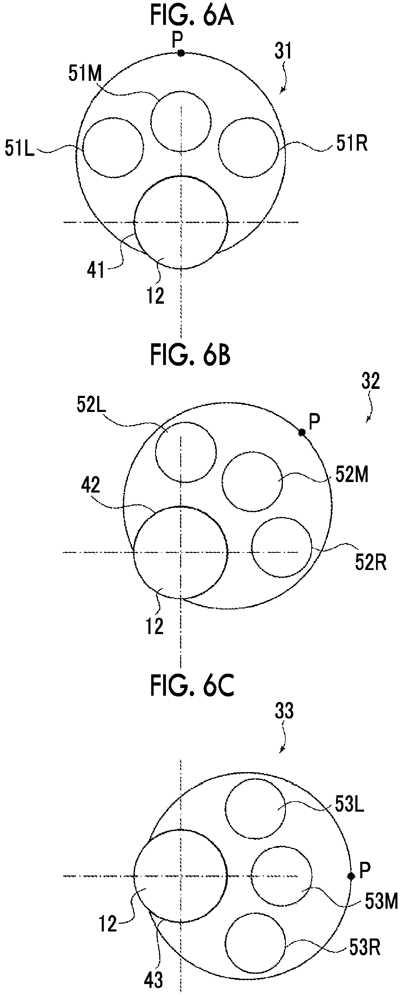

Figs. 5A and 5B are perspective views showing an example of a specific disposition aspect of the first to third guide ribs 31 to 33. Figs. 6A to 6C are views showing circumferential displacements of the first to third guide ribs 31 to 33 with respect to the inner tube 12.

In the present embodiment, as shown in Figs. 5A and 5B, and the like, the first to third guide ribs 31 to 33 are disposed to be displaced from each other in the circumferential direction with respect to the inner tube 12. "Being displaced from each other in the circumferential direction" means that the plurality of guide ribs 30 mounted on the inner tube 12 are not in the same orientation but are in orientations rotated about the inner tube 12, when viewed from the axial direction. In Figs. 6A to 6C, the first guide rib 31 and the second guide rib 32, and the second guide rib 32 and the third guide rib 33 are each in an orientation rotated by 45° about the inner tube 12.

In a case where the eccentric type guide rib 30 in which the tube attachment portion 40 is eccentrically disposed is mounted on the inner tube 12 and housed in the sheath 11, a contact pressure of the guide rib 30 against the sheath 11 is maximized at a point P that is farthest from the inner tube 12 when viewed from the axial direction. That is, the frictional force with the sheath 11 is maximized at the point P of the guide rib 30. In addition, in the guide rib 30, a portion where the guide portion 50 is provided protrudes from the inner tube 12 and is engaged with the colonic stent 2 mounted on the inner tube 12.

In the delivery system 100 to which the plurality of guide ribs 30 are applied, in a case where all the guide ribs 30 are disposed in the same orientation with respect to the inner tube 12, portions where the frictional forces between the sheath 11 and the guide ribs 30 are maximized are arranged in the axial direction at the same position in the circumferential direction. Therefore, the resistance force when pulling out the sheath 11 locally increases as viewed from the axial direction.

In addition, in a case where the colonic stent 2 is placed in the curved lesion site L, the catheter is inserted along an outer-side surface having a larger curvature radius. Therefore, in a case where the points P of the plurality of guide ribs 30 are arranged and located on an inner-side surface side having a smaller curvature radius, all engagements between the colonic stent 2 and the plurality of guide ribs 30 are likely to be released. Accordingly, when releasing the colonic stent 2 from the sheath 11, the increased frictional force between the sheath 11 and the colonic stent 2 on the outer-side surface side also makes it more likely for the colonic stent 2 to deviate with respect to the inner tube 12.

On the other hand, in a case where the plurality of guide ribs 30 are disposed to be displaced from each other in the circumferential direction with respect to the inner tube 12, portions where the frictional forces between the sheath 11 and the guide ribs 30 are maximized are at different positions in the circumferential direction. Therefore, the resistance force when pulling out the sheath 11 is dispersed in the circumferential direction as viewed from the axial direction. Accordingly, the sheath 11 can be smoothly pulled out as compared with a case where all the guide ribs 30 are disposed in the same orientation.

In addition, in the plurality of guide ribs 30, the portions that are engaged with the colonic stent 2 are different in the circumferential direction. Therefore, in a case where the colonic stent 2 is placed in the curved lesion site L, the possibility of all the guide ribs 30 and the colonic stent 2 being disengaged from each other is low. Accordingly, regardless of the shape of the lesion site L, the engagement between any one of the guide ribs 30 and the colonic stent 2 is held, so that it is possible to prevent the colonic stent 2 from deviating in the axial direction with respect to the inner tube 12 when releasing the colonic stent 2 from the sheath 11.

In addition, the adjacent guide ribs 30 are disposed such that a part of the guide portion 50 overlaps in the axial direction. Specifically, the middle guide portion 51M and the right guide portion 51R of the first guide rib 31 overlap with the left guide portion 52L and the middle guide portion 52M of the second guide rib 32 in the axial direction. Further, the middle guide portion 52M of the second guide rib 32 overlaps with the left guide portion 53L of the third guide rib 33 in the axial direction.

The first conversion means 20A is inserted only through the left guide portion 51L of the first guide rib 31. Since a portion of the second guide rib 32, which corresponds to the left guide portion 51L of the first guide rib 31, is blocked, it is possible to prevent erroneous operation when inserting the first conversion means 20A.

The second conversion means 20B is inserted through the middle guide portion 51M of the first guide rib 31 and the left guide portion 52L of the second guide rib 32. Since the middle guide portion 51M of the first guide rib 31 and the left guide portion 52L of the second guide rib 32 overlap with each other in the axial direction, the operation of removing the second conversion means 20B from the first guide rib 31 and the second guide rib 32 can be smoothly performed. In addition, the second conversion means 20B can be easily inserted through the first guide rib 31 and the second guide rib 32. Further, since a portion of the third guide rib 33, which corresponds to the left guide portion 52L of the second guide rib 32, is blocked, it is possible to prevent erroneous operation when inserting the second conversion means 20B.

The third conversion means 20C is inserted through the right guide portion 51R of the first guide rib 31, the middle guide portion 52M of the second guide rib 32, and the left guide portion 53L of the third guide rib 33. Since the right guide portion 51R of the first guide rib 31, the middle guide portion 52M of the second guide rib 32, and the left guide portion 53L of the third guide rib 33 overlap with each other in the axial direction, the operation of removing the third conversion means 20C from the first to third guide ribs 31 to 33 can be smoothly performed. In addition, the third conversion means 20C can be easily inserted through the first to third guide ribs 31 to 33.

As described above, the plurality of first to third conversion means 20A to 20C are provided to correspond to the first to third guide ribs 31 to 33. Consequently, the first to third conversion means 20A to 20C that have passed through the first to third guide ribs 31 to 33 are extracted to the outside of the colonic stent 2 as appropriate, and the first stent portion 2A to the third stent portion 2C to be restrained by the first to third conversion means 20A to 20C can be easily distinguished and restrained. In addition, in a case where the colonic stent 2 is placed in the indwelling target site, the first to third holding wires 22A to 22C can be pulled out along the axial direction of the inner tube 12, and it is possible to suppress the occurrence of bending in the inner tube 12 or the catheter 1.

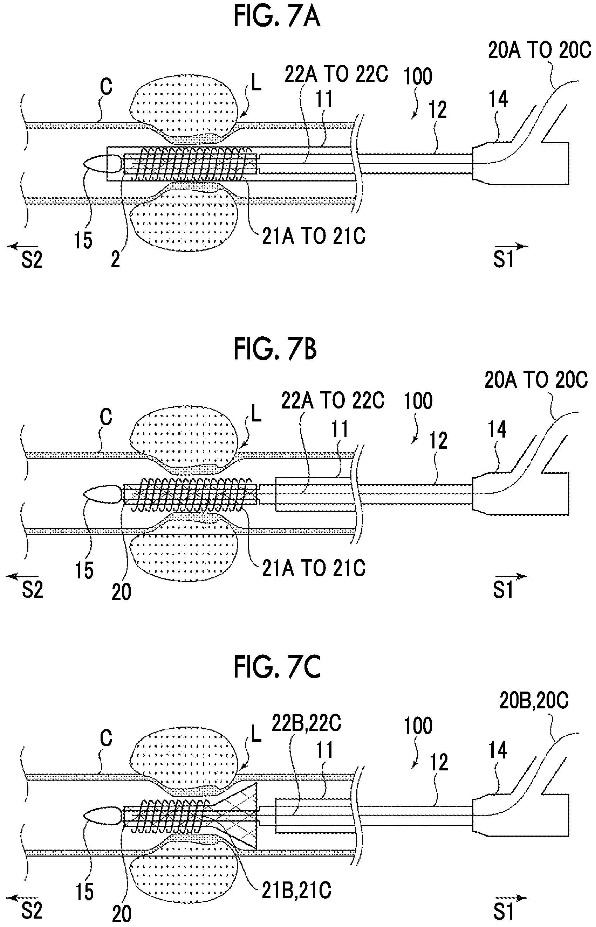

Figs. 7A to 7C are views showing a state change during placement of the colonic stent 2. In Figs. 7A to 7C, the colonic stent 2 is schematically shown, and the illustration of the detailed configurations of the first to third conversion means 20A to 20C is omitted. In addition, in Figs. 7A to 7C, the distal side S2 is an "oral side" and the proximal side S1 is an "anal side". Here, a case of expanding and placing the colonic stent 2 in order from the third stent portion 2C on the proximal side S1 will be described.

In a case of placing the colonic stent 2 in the lesion site L (indwelling target site) of the colon C, the sheath 11 and the inner tube 12 are inserted from the anal side along a guide wire (not shown) that is preemptively introduced into the colon C, and positioning is performed such that the colonic stent 2 is located at the lesion site L (refer to Fig. 7A).

Next, the sheath 11 is moved to the proximal side S1 (the anal side) in a positioned state, and the colonic stent 2 is released from the sheath 11 (refer to Fig. 7B). At this time, the colonic stent 2 is restrained by the first to third conversion means 20A to 20C and is maintained in a contracted state. In addition, the colonic stent 2 is restricted by the guide ribs 30 from deviating in the axial direction with respect to the inner tube 12 (the first guide rib 31 to the fourth guide rib 34). The first to fourth guide ribs 31 to 34 are configured as eccentric type guide ribs and are disposed in a state of being displaced from each other in the circumferential direction, so that the deviation of the colonic stent 2 in the axial direction with respect to the inner tube 12 can be reliably prevented. The colonic stent 2 may be released from the sheath 11 by moving the inner tube 12 to be pushed toward the oral side in a state in which the position of the sheath 11 is fixed.

When the entire colonic stent is expanded with the release from the sheath as in the conventional case, the position of the colonic stent is positioned by the expansion force of the colonic stent, making it difficult to adjust the indwelling position. In addition, the indwelling state of the colonic stent can be endoscopically confirmed at a time in point when the colonic stent is expanded in order from the distal side S2 to the proximal side S1, making it difficult to adjust the indwelling position during placement. On the other hand, in the present embodiment, since the colonic stent 2 is not expanded at a time in point when the colonic stent 2 is released from the sheath 11, the indwelling position of the colonic stent 2 can be adjusted while endoscopically confirming the position on the proximal side S1 in the state shown in Fig. 7B, making it easier to place the colonic stent 2 at an appropriate position. Moreover, the colonic stent 2 can also be rehoused in the sheath 11.

Next, for example, the first holding wire 22A is pulled out based on the operation of the pulling-out operation part 142 of the second operation part 14, and the first stent portion 2A of the colonic stent 2 is released from the restraint by the first conversion means 20A and is released. Only the first stent portion 2A transitions to the expanded state (refer to Fig. 7C). At this time, the second stent portion 2B and the third stent portion 2C are maintained in a contracted state by the second conversion means 20B and the third conversion means 20C. The first restraining strap 21A is pulled out and retrieved as appropriate.

Similarly, the second stent portion 2B and the third stent portion 2C of the colonic stent 2 are released to be put into an expanded state, and the colonic stent 2 is appropriately placed at the target indwelling position.

By applying the delivery system 100 to the indwelling technique of the colonic stent 2, the colonic stent 2 can be moderately expanded in various aspects partially in the axial direction according to the introduction direction of the catheter 1, the indwelling site, and the like. At this time, since the holding wire 22 as the conversion means 20 is held by the plurality of guide ribs 30, the holding wire 22 can be pulled out along the inner tube 12, and the occurrence of bending in the inner tube 12 or the catheter 1 can be suppressed. As a result, the colonic stent 2 can be accurately placed in the indwelling target site of the colon C.

As described above, the catheter 1 according to the first embodiment is a catheter for delivering the colonic stent 2 (tubular indwelling device) to be placed in the body lumen, and includes the sheath 11, the elongated inner tube 12 (axial member) that is configured to move inside the sheath 11 in the axial direction, the plurality of guide ribs 30 that are disposed on the inner tube 12 to be spaced apart from each other in the axial direction and are configured to position the colonic stent 2, which is in a radially contracted state inside the sheath 11, in the axial direction of the inner tube 12, and the restraining strap 21 and the holding wire 22 (linear member) that maintain axially at least a portion of the colonic stent 2 in a contracted state, with the colonic stent 2 released from the sheath 11. The plurality of guide ribs 30 each include the tube attachment portion 40 (attachment portion) that is eccentrically provided with respect to the circumscribed circle of the guide rib 30 in a cross-section orthogonal to the axial direction and to which the inner tube 12 is attached, and the guide portion 50 through which the restraining strap 21 and the holding wire 22 are inserted, and are disposed to be displaced from each other in the circumferential direction with respect to the inner tube 12.

By applying the eccentric type guide rib 30, the influence of the guide rib 30 on a filling rate inside the sheath 11 is reduced, and release properties when releasing the colonic stent 2 from the sheath 11 are improved. In addition, since the resistance force when pulling out the sheath 11 is dispersed in the circumferential direction as viewed from the axial direction, the sheath 11 can be smoothly pulled out as compared with a case where all the guide ribs 30 are disposed in the same orientation. Further, regardless of the shape of the lesion site L, the engagement between any one of the guide ribs 30 and the colonic stent 2 is held, so that it is possible to prevent the colonic stent 2 from deviating in the axial direction with respect to the inner tube 12 when releasing the colonic stent 2 from the sheath 11. Therefore, with the catheter 1 and the delivery system 100, the colonic stent 2 can be accurately placed at the lesion site L of the colon C, which is the indwelling target site.

In addition, in the catheter 1, the first guide rib 31 (one guide rib), among the first to third guide ribs 31 to 33 (a plurality of guide ribs), has the left guide portion 51L, the middle guide portion 51M, and the right guide portion 51R (a plurality of guide portions), and the second guide rib 32 and the third guide rib 33 (another guide rib) have circumferential displacement angles with respect to the first guide rib 31 defined based on the relative positional relationship between the left guide portion 51L, the middle guide portion 51M, and the right guide portion 51R (the plurality of guide portions of the one guide rib). The same applies to the displacement angles of the first guide rib 31 and the third guide rib 33 (another guide rib) with respect to the second guide rib 32 (one guide rib), and the displacement angles of the first guide rib 31 and the second guide rib 32 (another guide rib) with respect to the third guide rib 33 (one guide rib). As a result, the orientations of the first to third guide ribs 31 to 33 can be easily determined according to the insertion aspects of the plurality of conversion means 20 consisting of a set of the restraining strap 21 and the holding wire 22, for example, such that the plurality of conversion means 20 do not interfere with each other.

In addition, in the catheter 1, the second guide rib 32 (another guide rib) has the left guide portion 52L, the middle guide portion 52M, and the right guide portion 52R (a plurality of guide portions), and the first guide rib 31 (one guide rib) and the second guide rib 32 are disposed such that the middle guide portion 51M and the left guide portion 52L, and the right guide portion 51R and the middle guide portion 52M (one of the plurality of guide portions) overlap with each other in the axial direction, while there is no overlap in the left guide portion 51L and the right guide portion 52R (another guide portion) in the axial direction. Consequently, the plurality of conversion means 20 can be held in parallel, and the plurality of conversion means 20 can be prevented from interfering with each other. Further, the conversion means 20 including the restraining strap 21 and the holding wire 22 can be easily inserted through the guide portion 50 without causing an erroneous operation. Therefore, the operability when pulling out the conversion means 20 is improved.

In addition, the catheter 1 includes the first to third restraining straps 21A to 21C (a plurality of linear members) and the first to third holding wires 22A to 22C (a plurality of linear members), and the guide rib 30 has the same number of the guide portions 50L, 50M, and 50R as the number of the first to third restraining straps 21A to 21C and the number of the first to third holding wires 22A to 22C, and the number of the first to third restraining straps 21A to 21C and the first to third holding wires 22A to 22C to be inserted through the guide portions 50 increases as the guide rib 30 is disposed closer to the proximal side. As a result, the first to third restraining straps 21A to 21C and the first to third holding wires 22A to 22C can be individually inserted through and guided by the guide portions 50L, 50M, and 50R.

In addition, in the catheter 1, the plurality of guide ribs 30 have the same structure. As a result, components of the catheter 1 can be simplified, and product costs can be reduced.

Although the invention made by the inventors has been specifically described above based on the embodiment, the present invention is not limited to the above-described embodiment and can be changed without departing from the gist of the present invention.

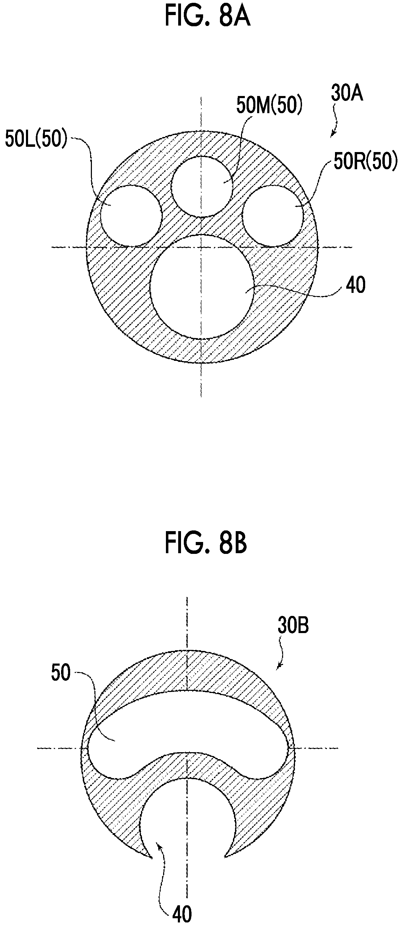

For example, the structure of the guide rib 30 described in the first embodiment is an example, and the present invention is not limited to this. The guide rib 30 can be freely changed as appropriate as long as the tube attachment portion 40 is formed eccentrically. For example, as shown in Fig. 8A, the tube attachment portion 40 may be formed inside a guide rib 30A as a through-hole.

In addition, for example, as shown in Fig. 8B, the guide portion 50 may be configured as one large through-hole through which the plurality of conversion means 20 can be inserted. In a case where a plurality of guide ribs 30B shown in Fig. 8B are disposed with respect to the inner tube 12, the displacement angle of another guide rib 30B with respect to one guide rib 30B is defined based on the size of the guide portion 50 formed in the guide rib 30B (for example, the length of at least a portion of the arc). As a result, the orientations of the plurality of guide ribs 30B can be easily determined according to the insertion aspects of the conversion means 20 consisting of the set of the restraining strap 21 and the holding wire 22, for example, such that the plurality of conversion means 20 do not interfere with each other.

In addition, for example, in the first embodiment, the first to third conversion means 20A to 20C having independent configurations are provided, but this is merely an example, and the present invention is not limited to this, and the restraining strap 21 and the holding wire 22 may be used in common. For example, the shape, the length, and the like of the restraining strap 21 or the holding wire 22 may be adjusted such that the colonic stent 2 is released in a predetermined order according to the operation amount or the operation mode. Further, the number of the conversion means is merely an example and is not limited to this, and at least one conversion means may be provided.

In addition, in the first embodiment, a case has been described in which the entire axial portion of the colonic stent 2 is maintained in a contracted state by the first to third conversion means 20A to 20C, but the conversion means 20 need only maintain at least a portion of the colonic stent 2 in a contracted state. For example, the second and third conversion means 20B and 20C may be configured to maintain, at the time of the release from the sheath 11, the second stent portion 2B and the third stent portion 2C of the colonic stent 2 in a contracted state, and the first stent portion 2A may be configured to be deployed with the release from the sheath 11.

In addition, in the first embodiment, the colonic stent 2 which is a straight type bare stent has been described as an example of the tubular indwelling device to be placed by the delivery system 100, but this is merely an example, and the present invention is not limited to this. For example, as the tubular indwelling device to be placed by the delivery system 100, a single-side or double-side flared type stent can be applied. Further, the tubular indwelling device may be a covered stent in which a membrane is disposed entirely or partially on the peripheral surface of the skeleton. However, in a case of the covered stent, in order to extract the conversion means 20 from the inside to the outside, a mesh type having pores is suitable for the membrane.

Furthermore, the present invention is not limited to the colonic stent 2 described in the embodiment and can be applied to a digestive tract stent that is placed in a digestive tract such as an esophagus or a colon, or a stent graft that is placed in a blood vessel. In this case, a fluid flowing through the digestive tract includes, for example, food immediately after ingestion before any digestion has occurred, a substance that has been broken down as food passes through the digestive tract, a substance that has not been digested even after passing through the digestive tract (for example, feces or the like), and the like, regardless of the state of the substance.

### [Second Embodiment]

In a second embodiment, as an example of the present invention, a catheter 1_2 and a delivery system 100_2 for placing a colonic stent 2_2, which is a tubular indwelling device, in the colon C in order to treat occlusion (stenosis) by pushing and expanding the lesion site L of the colon C (for example, an occluded part or a stenotic part of the colon) radially outward will be described.

A delivery device for placing the colonic stent 2_2 at the indwelling target site is referred to as a "catheter 1_2", and a system in which the colonic stent 2_2 is loaded into the catheter 1_2 is referred to as a "delivery system 100_2".

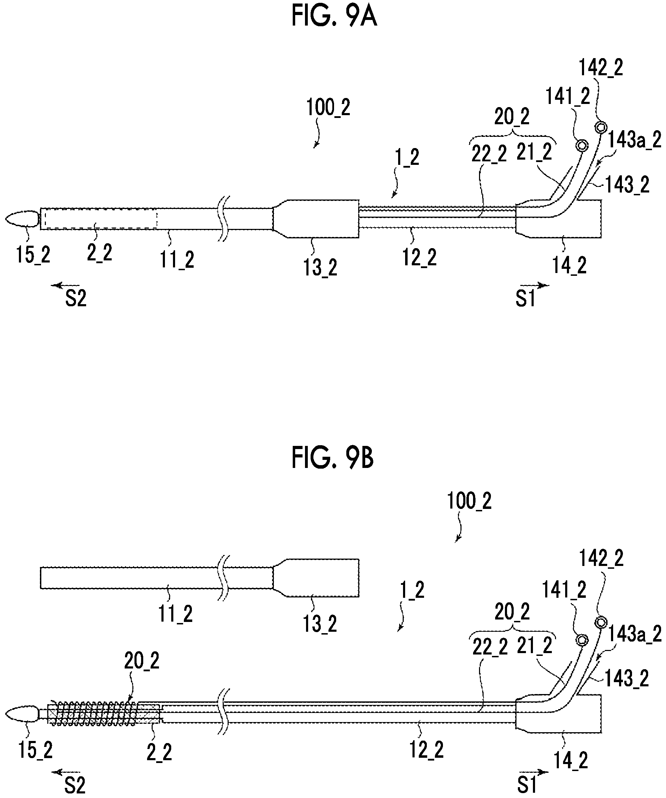

Figs. 9A and 9B are views showing a configuration of the delivery system 100_2. Fig. 9A shows a state in which the delivery system 100_2 is assembled, and Fig. 9B shows a state in which the delivery system 100_2 is disassembled. In Figs. 9A and 9B, in order to facilitate the understanding of the invention, a size (a length, a diameter dimension, and the like), a shape, or the like of each member constituting the delivery system 100_2 are schematically shown. In the following description, descriptions will be provided by representing the left side in Figs. 9A and 9B as the distal side S2 and the right side as the proximal side S1.

As shown in Figs. 9A and 9B, the delivery system 100_2 includes the catheter 1_2 and the colonic stent 2_2. The delivery system 100_2 is used, for example, endoscopically by being inserted into a forceps hole of an endoscope when placing the colonic stent 2_2 in the colon.

The colonic stent 2_2 has a tubular shape to define a tubular flow path through which a digestive substance flows. The colonic stent 2_2 is, for example, a bare stent that is formed of only the skeleton. The skeleton is a reinforcing member for maintaining an expanded state of the colonic stent 2_2 and is formed to be self-expandable from a contracted state in which the skeleton is contracted inward to an expanded state in which the skeleton is expanded outward, in a radial direction substantially orthogonal to an axial direction.

The skeleton is formed, for example, by weaving a wire material into a tubular shape. The skeleton is formed by, for example, weaving two wire materials that helically extend while being folded back in a zigzag shape (Z-like shape) at a predetermined pitch into a diamond-like mesh shape (fence-like shape) such that bending portions (one peak (a convex portion at one end side in the axial direction) and the other valley (a convex portion at the other end side in the axial direction) ) mesh with each other. In this case, in a state in which tension is applied to the colonic stent 2_2 in the axial direction, the bending portions of the wire materials forming the mesh closely intersect each other, so that elongation in the axial direction is restricted.

The skeleton is not limited to the above-described skeleton structure and may be configured as, for example, a helical skeleton in which a single metal wire material is helically wound while being bent in a zigzag shape (Z-like shape) such that a peak (a bending portion on a tip end side) and a valley (a bending portion on a rear end side) are alternately formed. In addition, the skeleton may be formed by performing laser processing on a metal cylindrical member. Further, the wire material forming the skeleton may be a plurality of wire materials.

Examples of the material forming the skeleton include known metals or metal alloys represented by stainless steel, a nickel-titanium alloy (nitinol), a titanium alloy, and the like. In addition, an alloy material having X-ray contrast properties may be used. In this case, the position of the colonic stent 2_2 can be confirmed from the outside of the body. The skeleton may be formed of a material other than the metal material (for example, ceramics, resins, or the like).

The material, the wire type (for example, a circular wire material such as wire, or angular wire material by laser cutting), the cross-sectional area (corresponding to the wire diameter in a case of a round wire material), and the number of bends and the bending shape in the circumferential direction (the number of peaks and the shape of the peak) of the wire material forming the skeleton, an interval between wire materials in the axial direction (skeleton amount per unit length), and the like are selected as appropriate, for example, based on criteria such as storage properties in the sheath required for the colonic stent 2_2 according to the indwelling site, release properties from the sheath, indwelling capability (corresponding to an expansion force), flexibility (ease of bending), and the like.

The catheter 1_2 includes a sheath 11_2, an inner tube 12_2, a first operation part 13_2, a second operation part 14_2, a distal end tip 15_2, a conversion means 20_2, and the like.

The sheath 11_2 is, for example, a tubular member formed of a material having flexibility. The sheath 11_2 is formed of, for example, a nylon resin and includes a low friction layer made of a high-density polyethylene to which a fluorine-based resin or silicon is added, or the like provided on the inner surface thereof in order to improve the release properties of the colonic stent 2_2.

The inner tube 12_2 is, for example, an elongated axial member formed of a material having moderate hardness and flexibility, such as a resin or a metal. The inner tube 12_2 is formed of, for example, a silicon resin. A proximal end of the sheath 11_2 is connected to the first operation part 13_2, and a proximal end of the inner tube 12_2 is connected to the second operation part 14_2. In addition, the distal end tip 15_2 is disposed at a distal end of the inner tube 12_2.

The inner tube 12_2 is inserted into the sheath 11_2 and is disposed to extend to a distal side S2 of the sheath 11_2. The sheath 11_2 and the inner tube 12_2 are configured to move relative to each other in the axial direction of the sheath 11_2 through the operations of the first operation part 13_2 and the second operation part 14_2.

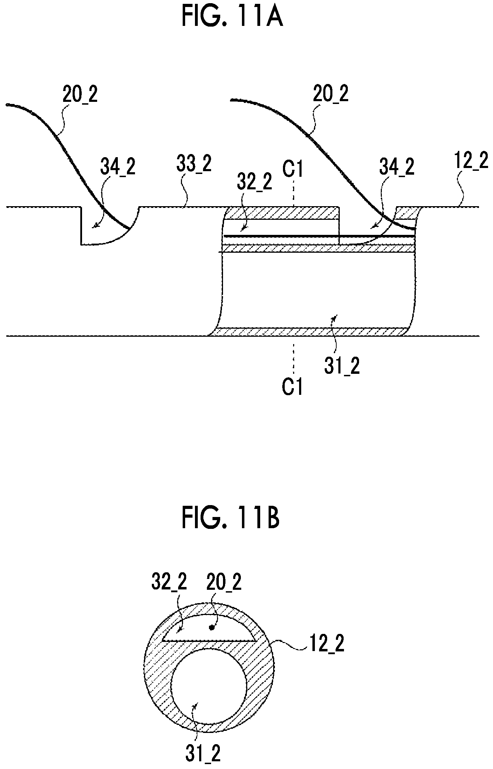

The colonic stent 2_2 is attached to an end part (a stent attachment portion 33_2, refer to Fig. 11A and the like) of the inner tube 12_2 on the distal side S2 and is housed in the sheath 11_2 in a radially expandable contracted state. Specifically, the colonic stent 2_2 is put into a contracted state by being radially folded while elongating in the axial direction, and is housed in the sheath 11_2. At this time, the colonic stent 2_2 is held in a contracted state by the conversion means 20_2.

The conversion means 20_2 is configured to maintain axially at least a portion (for example, a first stent portion 2A_2 and the like) of the colonic stent 2_2 in a contracted state, with the colonic stent 2_2 released from the sheath 11_2, and to convert the portion from a contracted state to an expanded state.

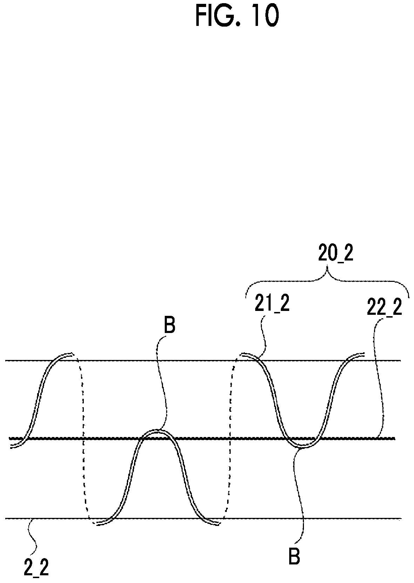

Fig. 10 is a schematic view showing an example of the conversion means 20_2. As shown in Fig. 10, the conversion means 20_2 is composed of a restraining strap 21_2 wound around an outer peripheral surface of the colonic stent 2_2 and a holding wire 22_2 engaged with the restraining strap 21_2. The conversion means 20_2 is attached to, for example, the colonic stent 2_2 mounted on the inner tube 12_2 such that the colonic stent 2_2 is radially contracted.

The restraining strap 21_2 and the holding wire 22_2 are formed of, for example, a material having a predetermined strength and rigidity. As the restraining strap 21_2 and the holding wire 22_2, for example, a sewing thread such as a nylon fiber or a fluorine fiber, a metal wire made of a nickel-titanium alloy or stainless steel, or a strap-like member made of a resin can be applied. It is preferable that the restraining strap 21_2 and the holding wire 22_2 are formed of different materials in order to improve the slipperiness in a case of being pulled out.

The colonic stent 2_2 is held in a contracted state by a portion of the restraining strap 21_2 and the holding wire 22_2 on the distal side S2. End parts of the restraining strap 21_2 and the holding wire 22_2 on the proximal side S1 are each extracted from, for example, a branch port 143a_2 on one side of a Y connector 143_2 of the second operation part 14_2 and are connected to pulling-out operation portions 141_2 and 142_2. The pulling-out operation portions 141_2 and 142_2 can be pulled in a predetermined direction (for example, in an upper right direction in Fig. 9A, or the like) so that the restraining strap 21_2 and the holding wire 22_2 can be pulled out. A dial (not shown) may be provided on the second operation part 14_2, and the holding wire 22_2 may be pulled out while making fine adjustments through a rotational operation of the dial, or the like.

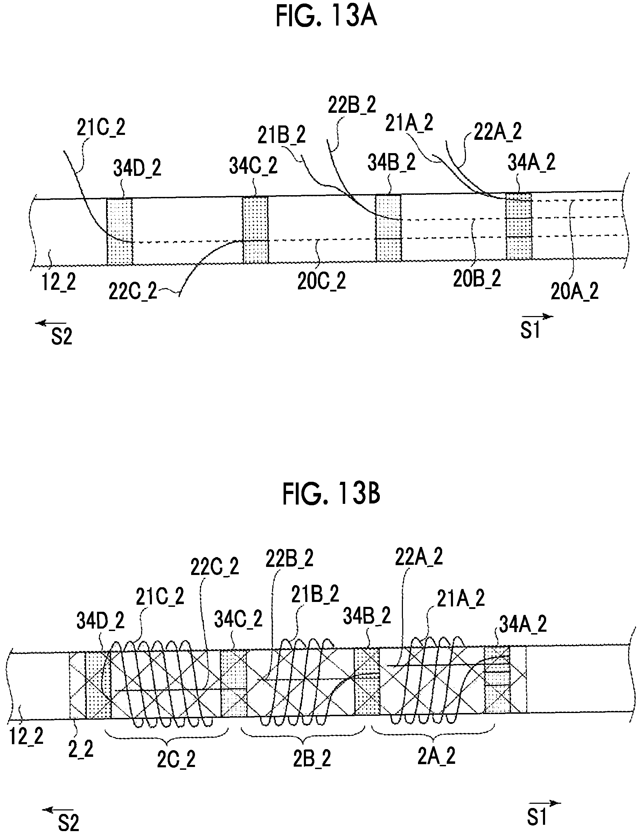

In the example shown in Fig. 10, the restraining strap 21_2 is wound around an outer peripheral surface of a portion (for example, a third stent portion 2C_2 or the like shown in Fig. 13B) of the colonic stent 2_2 such that the restraining strap 21_2 bends and reverses a winding direction thereof for each turn, for example. Meanwhile, the holding wire 22_2 is disposed along the axial direction of the colonic stent 2_2 and is engaged with a bending portion B formed at the restraining strap 21_2. That is, the restraining strap 21_2 is wound in an aspect in which the restraining strap 21_2 cannot hold a wound state by itself, and is held in a non-detachable manner by being engaged with the holding wire 22_2. Therefore, in a case where the engagement between the restraining strap 21_2 and the holding wire 22_2 is released, the restraining strap 21_2 is naturally detached from the colonic stent 2_2. As a result, a portion of the colonic stent 2_2 is released from the contracted state and transitions to the expanded state.

Figs. 11A and 11B are schematic views showing a structure of the inner tube 12_2 according to the embodiment. Fig. 11A is a partial longitudinal sectional view of the inner tube 12_2 as viewed from the side, and Fig. 11B is a transverse sectional view (a cross-sectional view taken along C1-C1 of Fig. 11A) of the inner tube 12_2 as viewed from the axial direction.

As shown in Figs. 11A and 11B, the inner tube 12_2 includes a first guide portion 31_2 and a second guide portion 32_2. In addition, the inner tube 12_2 includes the stent attachment portion 33_2 on the outer peripheral surface thereof on the distal side S2 in the axial direction.

The inner tube 12_2 is, for example, an integral multi-lumen tube formed by extrusion molding. The first guide portion 31_2 and the second guide portion 32_2 are composed of two lumens that are formed separately.

The first guide portion 31_2 is, for example, a through-hole having a circular cross-section as viewed from the axial direction. A guide wire (not shown) is inserted through the first guide portion 31_2.

The second guide portion 32_2 is, for example, a through-hole having an arch-shaped cross-section as viewed from the axial direction. The conversion means 20_2 is inserted through the second guide portion 32_2.

The stent attachment portion 33_2 is provided with an extraction portion 34_2 for extracting the conversion means 20_2 inserted through the second guide portion 32_2. The extraction portion 34_2 is formed, for example, by partially cutting and notching the outer peripheral surface of the inner tube 12_2. In addition, for example, the extraction portion 34_2 may be a through-hole through which the second guide portion 32_2 and the outside communicate with each other.

The shapes and the sizes of the first guide portion 31_2 and the second guide portion 32_2 are not particularly limited. The first guide portion 31_2 need only be shaped and sized to allow the guide wire (not shown) to smoothly advance and retreat, and the second guide portion 32_2 need only be shaped and sized to allow the conversion means 20_2 to smoothly advance and retreat. In addition, in the examples shown in Figs. 11A and 11B, the first guide portion 31_2 is provided eccentrically with respect to the outer shape of the inner tube 12_2, but may be provided concentrically. Further, in a case where a plurality of the conversion means 20_2 are used, a plurality of the second guide portions 32_2 that are spatially separated from each other may be provided according to the number of conversion means 20_2, and the plurality of conversion means 20_2 may be configured to be individually guided.

The colonic stent 2_2 is disposed on the stent attachment portion 33_2 in a state of being radially contracted inside the sheath 11_2 and being positioned in the axial direction. The stent attachment portion 33_2 has a surface structure capable of suppressing the movement of the colonic stent 2_2 in the axial direction when the colonic stent 2_2 is released from the sheath 11_2. Unlike the conventional positional deviation prevention structure, the ribs are not provided on the outer peripheral surface of the inner tube 12_2, so that the filling rate inside the sheath 11_2 is not increased.

That is, the stent attachment portion 33_2 has a greater frictional force against the colonic stent 2_2 as compared with a frictional force of each of the other members (for example, the sheath 11_2) in the catheter 1_2. Specifically, the frictional force between the stent attachment portion 33_2 and the colonic stent 2_2 is greater than the frictional force between the sheath 11_2 and the colonic stent 2_2. For example, by applying a coating made of an elastic resin material such as silicone rubber, urethane rubber, or nylon rubber to the outer peripheral surface of the stent attachment portion 33_2, the frictional force between the stent attachment portion 33_2 and the colonic stent 2_2 can be increased. In addition, for example, the outer peripheral surface of the stent attachment portion 33_2 may be covered with a tube molded of the elastic resin material. Since the low friction layer made of a fluorine-based resin or the like is provided on the inner surface of the sheath 11_2, the coating or the tube made of an elastic resin material is provided on the stent attachment portion 33_2, thereby reliably increasing the frictional force between the stent attachment portion 33_2 and the colonic stent 2_2 to be greater than the frictional force between the sheath 11_2 and the colonic stent 2_2.

The coating or the tube for increasing the frictional force may be provided to cover the entire surface of the stent attachment portion 33_2 or may be provided such that a region where no coating or tube is provided is partially formed in the axial direction and the circumferential direction. Moreover, the stent attachment portion 33_2 may be subjected to a roughening treatment to increase the frictional force through minute irregularities.

In addition, it is preferable that the frictional force between the stent attachment portion 33_2 and the colonic stent 2_2 is greater than the frictional force between the inner surface of the sheath 11_2 and the conversion means 20_2. That is, it is preferable that the frictional force between the stent attachment portion 33_2 and the colonic stent 2_2 is greater than the frictional force generated by the friction between the sheath 11_2 and the other members when releasing the colonic stent 2_2 from the sheath 11_2, specifically, the frictional force generated between the inner surface of the sheath 11_2 and the colonic stent 2_2 and the frictional force generated between the inner surface of the sheath 11_2 and the conversion means 20_2. As a result, when releasing the colonic stent 2_2 from the sheath 11_2, it is possible to suppress the colonic stent 2_2 from being pulled by the pulling-out operation of the sheath 11_2 and moving in the axial direction.

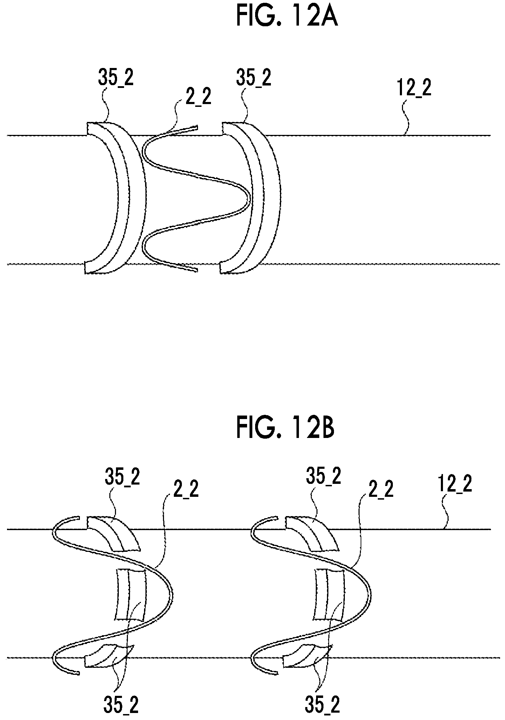

Further, as shown in Figs. 12A and 12B, a protruding rib 35_2 that protrudes radially outward is provided on the outer peripheral surface of the stent attachment portion 33_2. The protruding rib 35_2 is fixed to the outer peripheral surface of the inner tube 12_2 (stent attachment portion 33_2) by, for example, adhesion, welding, or the like. The protruding rib 35_2 physically restricts the movement of the colonic stent 2_2 in the axial direction when the colonic stent 2_2 is released from the sheath 11_2.

A height (thickness) of the protruding rib 35_2 is smaller than the outer shape of the circumscribed circle of the colonic stent 2_2 during contraction. That is, the protruding rib 35_2 is configured not to protrude radially outward more than the skeleton of the colonic stent 2_2 when the colonic stent 2_2 is mounted on the stent attachment portion 33_2 and contracted. Since the filling rate inside the sheath 11_2 is not increased even in a case where the protruding rib 35_2 is provided, the provision of the protruding rib 35_2 does not impair the operability when pulling out the sheath 11_2.

The protruding rib 35_2 is disposed to enter a gap in the skeleton of the colonic stent 2_2 when the colonic stent 2_2 is mounted on the stent attachment portion 33_2 and contracted.

For example, as shown in Fig. 12A, a plurality of the protruding ribs 35_2 are arranged in the axial direction. In Fig. 12A, the protruding rib 35_2 having an annular shape is disposed at two locations in the axial direction of the inner tube (stent attachment portion 33_2). A spacing between the two protruding ribs 35_2 axially adjacent to each other is set to be, for example, the same as a length between the adjacent bending portions of the colonic stent 2_2.

The colonic stent 2_2 is disposed such that the skeleton of the colonic stent 2_2 is partially interposed between the protruding ribs 35_2 axially adjacent to each other. The bending portion of the colonic stent 2_2 is close to the protruding rib 35_2, and in a case where the colonic stent 2_2 attempts to move in the axial direction, the outer side (tensile side) of the bending portion comes into contact with the protruding rib 35_2. Therefore, the movement of the colonic stent 2_2 in the axial direction when the colonic stent 2_2 is released from the sheath 11_2 is physically restricted.

In addition, for example, as shown in Fig. 12B, a plurality of protruding ribs 35_2 may be provided along the circumferential direction on the inner tube 12_2. In Fig. 12B, the plurality of protruding ribs 35_2 having an annular shape as a whole are disposed at two locations in the axial direction of the inner tube 12_2 (stent attachment portion 33_2). A spacing between two protruding ribs 35_2 adjacent to each other in the circumferential direction need only be larger than, for example, an outer diameter of the wire material forming the skeleton of the colonic stent 2_2.

The colonic stent 2_2 is disposed such that the skeleton of the colonic stent 2_2 enters a space between the protruding ribs 35_2 adjacent to each other in the circumferential direction, and the bending portion of the colonic stent 2_2 is disposed to wrap around the protruding ribs 35_2. The bending portion of the colonic stent 2_2 is close to the protruding rib 35_2, and in a case where the colonic stent 2_2 attempts to move in the axial direction, the inner side (compression side) of the bending portion is locked to the protruding rib 35_2. Therefore, the movement of the colonic stent 2_2 in the axial direction when the colonic stent 2_2 is released from the sheath 11_2 is physically restricted.

Figs. 13A and 13B are schematic views showing an attachment aspect of the colonic stent 2_2 and the conversion means 20_2 with respect to the inner tube 12_2.

As shown in Figs. 13A and 13B, first to third conversion means 20A_2 to 20C_2 are provided to correspond to the first stent portion 2A_2 to the third stent portion 2C_2 in which the colonic stent 2_2 is divided into three in the axial direction. The first stent portion 2A_2 is a portion located on the most proximal side S1, the third stent portion 2C_2 is a portion located on the most distal side S2, and the second stent portion 2B_2 is a portion between the first stent portion 2A_2 and the second stent portion 2B_2.

The first to third conversion means 20A_2 to 20C_2 include first to third restraining straps 21A_2 to 21C_2 and first to third holding wires 22A_2 to 22C_2, respectively. The first to third conversion means 20A_2 to 20C_2 are each configured to independently operate, and the expansion of a stent portion to be expanded (for example, the first stent portion 2A_2 or the like), among the first to third stent portions 2A_2 to 2C_2 of the colonic stent 2_2, can be flexibly controlled while confirming the indwelling state or the like.

First to fourth extraction portions 34A_2 to 34D_2 are disposed in the inner tube 12_2 to be spaced apart from each other in order from the proximal side S1 along the axial direction. The first stent portion 2A_2 of the colonic stent 2_2 is located between the first extraction portion 34A_2 and the second extraction portion 34B_2, the second stent portion 2B_2 is located between the second extraction portion 34B_2 and the third extraction portion 34C_2, and the third stent portion 2C_2 is located between the third extraction portion 34C_2 and the fourth extraction portion 34D_2.

The first conversion means 20A_2 is extracted from the first extraction portion 34A_2 on the most proximal side S1. The first holding wire 22A_2 of the first conversion means 20A_2 is extracted to the outside of the colonic stent 2_2 from the first extraction portion 34A_2 and is disposed along the first stent portion 2A_2. Similar to the first holding wire 22A_2, the first restraining strap 21A_2 is extracted to the outside of the colonic stent 2_2 from the first extraction portion 34A_2 and is wound around the outer peripheral surface of the first stent portion 2A_2 from the proximal side S1 toward the distal side S2.

The second conversion means 20B_2 passes through the first extraction portion 34A_2 and is re-inserted through the second guide portion 32_2 of the inner tube 12_2, and is extracted from the second extraction portion 34B_2. The second holding wire 22B_2 of the second conversion means 20B_2 is extracted to the outside of the colonic stent 2_2 from the second extraction portion 34B_2 and is disposed along the second stent portion 2B_2. Similar to the second holding wire 22B_2, the second restraining strap 21B_2 is extracted to the outside of the colonic stent 2_2 from the second extraction portion 34B_2 and is wound around the outer peripheral surface of the second stent portion 2B_2 from the proximal side S1 toward the distal side S2.

The third conversion means 20C_2 passes through the first extraction portion 34A_2 and the second extraction portion 34B_2 and is re-inserted through the second guide portion 32_2 of the inner tube 12_2. The third holding wire 22C_2 of the third conversion means 20C_2 is extracted to the outside of the colonic stent 2_2 from the third extraction portion 34C_2 and is disposed along the third stent portion 2C_2. The third restraining strap 21C_2 passes through the third extraction portion 34C_2 and is further inserted through the second guide portion 32_2 of the inner tube 12_2. The third restraining strap 21C_2 is extracted from the fourth extraction portion 34D_2, folded back, and wound around the outer peripheral surface of the third stent portion 2C_2 from the distal side S2 toward the proximal side S1.

The third restraining strap 21C_2 does not necessarily need to be extracted from the fourth extraction portion 34D_2, and may be extracted from the third extraction portion 34C_2 together with the third holding wire 22C_2 and may be wound around the outer peripheral surface of the third stent portion 2C_2 from the proximal side S1 toward the distal side S2. In this case, the fourth extraction portion 34D_2 does not necessarily need to be disposed.

In addition, it is preferable that the end surface of the fourth extraction portion 34D_2 on which the third restraining strap 21C_2 is folded back is chamfered. Consequently, when retrieving the third restraining strap 21C_2 folded back on the proximal side S1, kinks or damage occurring on the third restraining strap 21C_2 due to friction with the end surface of the fourth extraction portion 34D_2 can be reduced.

Further, in the inner tube 12_2, the excess third restraining strap 21C_2 may be configured to be stored by forming the second guide portion 32_2 up to a portion that is located on the distal side S2 with respect to the fourth extraction portion 34D_2. In addition, the distal end of the third holding wire 22C_2 may also be stored in the second guide portion 32_2 located on the distal side S2. Similarly, the distal ends of the first holding wire 22A_2 and the second holding wire 22B_2 may each be stored in the second guide portion 32_2 located on the distal side S2. As a result, it is possible to prevent the distal ends of the first to third holding wires 22A_2 to 22C_2 from being stuck into the wall of the body lumen.

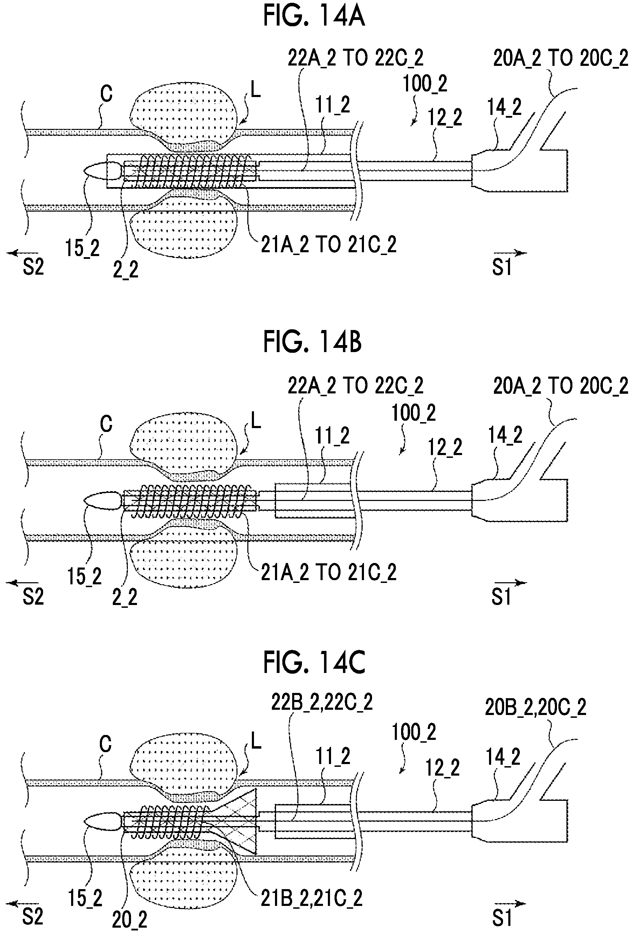

Figs. 14A to 14C are views showing a state change of the colonic stent 2_2 during the placement. In Figs. 14A to 14C, the colonic stent 2_2 is schematically shown, and the detailed configurations of the first to third conversion means 20A_2 to 20C_2 is not shown. In addition, in Figs. 14A to 14C, the distal side S2 is an "oral side" and the proximal side S1 is an "anal side". Here, a case of expanding and placing the colonic stent 2_2 in order from the first stent portion 2A_2 on the proximal side S1 will be described.

In a case of placing the colonic stent 2_2 in the lesion site L (indwelling target site) of the colon C, the sheath 11_2 and the inner tube 12_2 are inserted from the anal side along a guide wire (not shown) that is preemptively introduced into the colon C, and positioning is performed such that the colonic stent 2_2 is located at the lesion site L (refer to Fig. 14A).

Next, the sheath 11_2 is moved to the proximal side S1 (the anal side) in a positioned state, and the colonic stent 2_2 is released from the sheath 11_2 (refer to Fig. 14B) . At this time, the colonic stent 2_2 is restrained by the first to third conversion means 20A_2 to 20C_2 and is maintained in a contracted state. In addition, the deviation of the colonic stent 2_2 in the axial direction with respect to the inner tube 12_2 is restricted by the surface structure of the stent attachment portion 33_2 and the protruding rib 35_2 (refer to Fig. 12A).

Specifically, since the frictional force between the stent attachment portion 33_2 of the inner tube 12_2 and the colonic stent 2_2 is greater than the frictional force between the sheath 11_2 and the colonic stent 2_2, the colonic stent 2_2 is likely to be retained by the stent attachment portion 33_2 rather than to follow the movement of the sheath 11_2. In addition, the movement of the colonic stent 2_2 in the axial direction is physically restricted by the protruding rib 35_2. Therefore, it is possible to reliably prevent the colonic stent 2_2 from deviating in the axial direction with respect to the inner tube 12_2.

The colonic stent 2_2 may be released from the sheath 11_2 by moving the inner tube 12_2 to be pushed toward the distal side S2 (oral side) in a state in which the position of the sheath 11_2 is fixed.

When the entire colonic stent is expanded with the release from the sheath as in the conventional case, the position of the colonic stent is positioned by the expansion force of the colonic stent, making it difficult to adjust the indwelling position. In addition, the indwelling state of the colonic stent can be endoscopically confirmed at a time in point when the colonic stent is expanded in order from the distal side S2 to the proximal side S1, making it difficult to adjust the indwelling position during placement. On the other hand, in the present embodiment, since the colonic stent 2_2 is not expanded at a time in point when the colonic stent 2_2 is released from the sheath 11_2, the indwelling position of the colonic stent 2_2 can be adjusted while endoscopically confirming the position on the proximal side S1 in the state shown in Fig. 14B, making it easier to place the colonic stent 2_2 at an appropriate position. In addition, the colonic stent 2_2 can be rehoused in the sheath 11_2.

Next, for example, the first holding wire 22A_2 is pulled out based on the operation of the pulling-out operation portion 142_2 of the second operation part 14_2, and the first stent portion 2A_2 of the colonic stent 2_2 is released from the restraint by the first conversion means 20A_2 and is released. Only the first stent portion 2A_2 transitions to the expanded state (refer to Fig. 14C). At this time, the second stent portion 2B_2 and the third stent portion 2C_2 are maintained in a contracted state by the second conversion means 20B_2 and the third conversion means 20C_2. The first restraining strap 21A_2 is pulled out and retrieved as appropriate.

When pulling out the first holding wire 22A_2 to expand the colonic stent 2_2, a force also acts on the colonic stent 2_2 in the pulling-out direction, but the frictional force generated between the inner tube 12_2 and the colonic stent 2_2 acts as resistance. In addition, the skeleton of the colonic stent 2_2 in a contracted state is caught on the protruding rib 35_2 and physically locked. Therefore, since the position of the colonic stent 2_2 in the axial direction with respect to the inner tube 12_2 is held, the colonic stent 2_2 can be moved in conjunction with the inner tube 12_2, and the indwelling position of the colonic stent 2_2 can be moderately adjusted.

Similarly, the second stent portion 2B_2 and the third stent portion 2C_2 of the colonic stent 2_2 are released to be put into an expanded state, and the colonic stent 2_2 is appropriately placed at the target indwelling position.

By applying the delivery system 100_2 to the indwelling technique of the colonic stent 2_2, the colonic stent 2_2 can be moderately expanded in various aspects partially in the axial direction according to the introduction direction of the catheter 1_2, the indwelling site, and the like. At this time, since the holding wire 22_2 as the conversion means 20_2 is inserted through the second guide portion 32_2 formed inside the inner tube 12_2, the holding wire 22_2 can be smoothly pulled out. That is, it is possible to suppress the bending of the inner tube 12_2 or the catheter 1_2 due to the tension generated in the holding wire 22_2, and it is possible to accurately place the colonic stent 2_2 at the indwelling target site of the colon C.

As described above, the catheter 1_2 according to the second embodiment is the catheter 1_2 for delivering the colonic stent 2_2 (tubular indwelling device) to be placed in the colon C (body lumen), and includes the sheath 11_2, the inner tube 12_2 (elongated axial member) that is configured to move inside the sheath 11_2 in the axial direction and that includes the first guide portion 31_2 through which the guide wire is inserted, and the restraining strap 21_2 and the holding wire 22_2 (linear member) that maintain axially at least a portion of the colonic stent 2_2 in a contracted state, with the colonic stent 2_2 released from the sheath 11_2. The inner tube 12_2 includes the stent attachment portion 33_2 (positioning portion) that is provided on a portion of the inner tube 12_2 in the axial direction and that is disposed in a state in which the colonic stent 2_2, which is in a radially contracted state in the sheath 11_2, is positioned in the axial direction, and a second guide portion 32_2 through which the restraining strap 21_2 and the holding wire 22_2 are inserted.

With the catheter 1_2, the positioning portion is provided on a portion of the inner tube 12_2 in the axial direction, so that it is possible to prevent the colonic stent 2_2 from deviating in the axial direction with respect to the inner tube 12_2 when the colonic stent 2_2 is released from the sheath 11_2. Since a portion of the inner tube 12_2 functions as the positioning portion without forming the positioning portion using another member such as a rib, the operability when releasing the colonic stent 2_2 from the sheath 11_2 is ensured without increasing the filling rate inside the sheath 11_2. In addition, it is also suitable for achieving a decrease in diameter of the catheter 1_2.

In addition, even in a state in which the sheath 11_2 is released, axially at least a portion of the colonic stent 2_2 is maintained in a contracted state by the restraining strap 21_2 and the holding wire 22_2 and is gradually expanded with the pulling out of the holding wire 22_2, so that the colonic stent 2_2 can be placed at an indwelling target position while being partially expanded in various aspects according to the introduction direction of the catheter 1_2, the indwelling site, and the like. Further, since the holding wire 22_2 is inserted through the second guide portion 32_2 provided in the inner tube 12_2, the holding wire 22_2 can be pulled out along the inner tube 12_2, and it is possible to suppress the occurrence of bending in the inner tube 12_2 or the catheter 1_2.

Therefore, the colonic stent 2_2 is positioned with respect to the inner tube 12_2 without increasing the filling rate inside the sheath 11_2, so that the positional deviation of the colonic stent 2_2 with respect to the inner tube 12_2 can be prevented while ensuring the operability when releasing the colonic stent 2_2 from the sheath 11_2, and the colonic stent 2_2 can be accurately placed at the indwelling target site in the colon C.

In addition, in the catheter 1_2, the second guide portion 32_2 includes a through-hole (guide hole) provided inside the inner tube 12_2 (axial member) along the axial direction. The restraining strap 21_2 and the holding wire 22_2 are inserted through the guide hole to be aligned along substantially the entire length of the inner tube 12_2, and an advancing and retreating direction is restricted by the guide hole. Therefore, when pulling out the holding wire 22_2 to expand the colonic stent 2_2, the holding wire 22_2 can be pulled out to be aligned along the inner tube 12_2, and the effect of suppressing the occurrence of bending in the inner tube 12_2 or the catheter 1_2 is enhanced.

In addition, in the catheter 1_2, the inner tube 12_2 (axial member) further includes the extraction portion 34_2 for extracting the restraining strap 21_2 and the holding wire 22_2 (linear member) inserted through the through-hole (guide hole) to the outside of the inner tube 12_2. Since an extraction position of the restraining strap 21_2 and the holding wire 22_2 from the guide hole is determined by the extraction portion 34_2, the restraining strap 21_2 and the holding wire 22_2 can be easily extracted from a desired position corresponding to a restraint aspect of the colonic stent 2_2, and the colonic stent 2_2 can be restrained in a desired aspect.

In addition, in the catheter 1_2, the stent attachment portion 33_2 (positioning portion) has a greater frictional force against the colonic stent 2_2 (tubular indwelling device) as compared with the frictional force of each of the other members (for example, the sheath 11_2) different from the inner tube 12_2 (axial member). As a result, upon release from the sheath 11_2, the colonic stent 2_2 can be retained by the frictional force between the stent attachment portion 33_2 and the colonic stent 2_2, and the positional deviation of the colonic stent 2_2 in the axial direction with respect to the inner tube 12_2 can be suppressed.

In addition, in the catheter 1_2, the stent attachment portion 33_2 (positioning portion) includes the protruding rib 35_2 (protruding portion) that protrudes radially outward from the outer peripheral surface of the inner tube 12_2 (axial member) and that is provided to be axially arranged with the skeleton of the colonic stent 2_2 (tubular indwelling device). Since the movement of the colonic stent 2_2 in the axial direction is physically restricted by the protruding rib 35_2, the positional deviation of the colonic stent 2_2 in the axial direction with respect to the inner tube 12_2 upon release from the sheath 11_2 can be reliably suppressed.

Additionally, in the catheter 1_2, the plurality of protruding ribs 35_2 (protruding portions) are provided along the circumferential direction on the inner tube 12_2 (axial member), and the skeleton of the colonic stent 2_2 can be disposed between the plurality of protruding ribs 35_2. By mounting the colonic stent 2_2 such that the skeleton of the colonic stent 2_2 is disposed between the protruding ribs 35_2, the movement of the colonic stent 2_2 in the axial direction is physically restricted. Further, by ensuring that the protruding ribs 35_2 and the skeleton of the colonic stent 2_2 do not overlap with each other, it is possible to suppress an increase in the filling rate inside the sheath 11_2 due to the provision of the protruding ribs 35_2.

### [Modification Example 1]

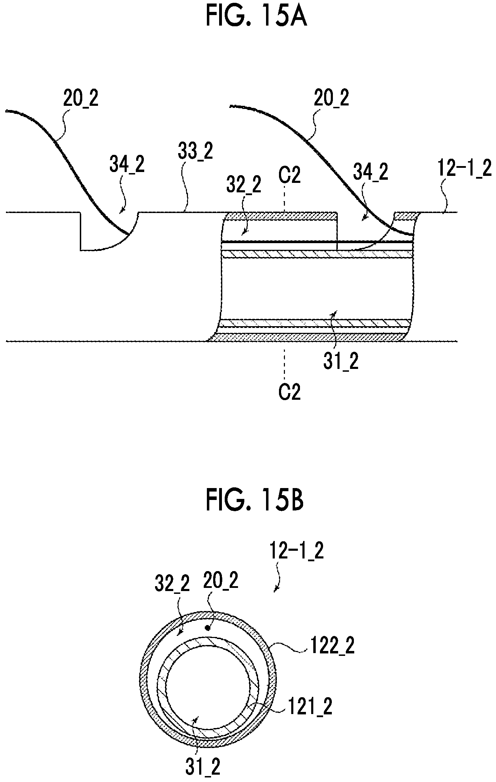

Figs. 15A and 15B are schematic views showing an example of an inner tube 12-1_2 according to Modification Example 1 of the second embodiment. Fig. 15A is a partial longitudinal sectional view of the inner tube 12-1_2 as viewed from the side, and Fig. 15B is a transverse sectional view of the inner tube 12-1_2 as viewed from the axial direction (a cross-sectional view taken along C2-C2 of Fig. 15A). The same configurations as those of the above-described embodiment can be applied to the components (for example, the protruding rib 35_2 and the like) other than the inner tube 12-1_2.

The inner tube 12_2 described in the embodiment is configured as a single molded product having two lumens. On the other hand, the inner tube 12-1_2 shown in Figs. 15A and 15B have a double tube structure consisting of a first tubular member 121_2 and a second tubular member 122_2.

An inner diameter of the second tubular member 122_2 disposed on the outer side is larger than an outer diameter of the first tubular member 121_2 disposed on the inner side. An inner space of the first tubular member 121_2 forms the first guide portion 31_2, and a space between the first tubular member 121_2 and the second tubular member 122_2 forms the second guide portion 32_2. The outer diameter of the first tubular member 121_2 and the inner diameter of the second tubular member 122_2 are set, for example, such that the conversion means 20_2 can smoothly advance and retreat in the second guide portion 32_2. The first tubular member 121_2 and the second tubular member 122_2 may be fixed to each other by adhesion, welding, or the like or may be simply loosely fitted to each other.

The inner tube 12-1_2 can be easily produced as compared with the inner tube 12_2 consisting of the integral multi-lumen tube described in the embodiment, because the inner tube 12-1_2 can be produced by combining the first tubular member 121_2 and the second tubular member 122_2. In addition, since cutting any portion in the circumferential direction leads to the second guide portion 32_2, the extraction portion 34_2 can be easily formed.

### [Modification Example 2]

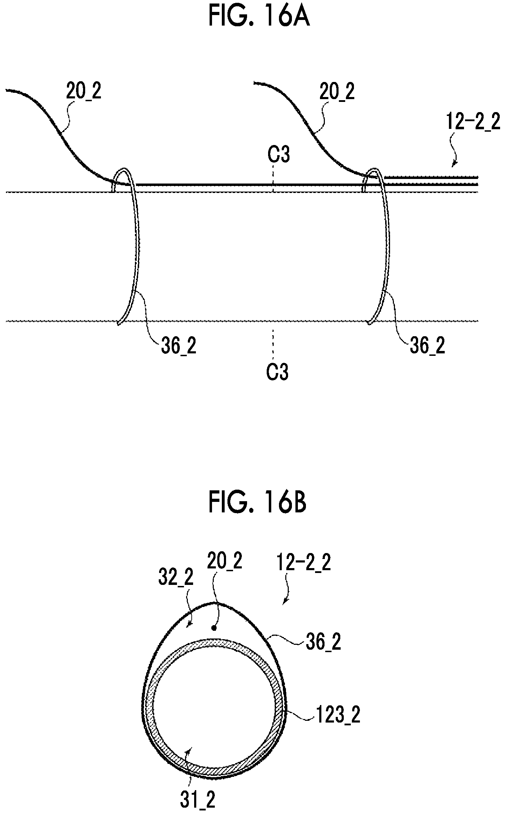

Figs. 16A and 16B are schematic views showing an example of an inner tube 12-2_2 according to Modification Example 2 of the second embodiment. Fig. 16A is a partial longitudinal sectional view of the inner tube 12-2_2 as viewed from the side, and Fig. 16B is a transverse sectional view of the inner tube 12-2_2 as viewed from the axial direction (a cross-sectional view taken along C3-C3 of Fig. 16A). The same configurations as those of the above-described embodiment can be applied to the components (for example, the protruding rib 35_2 and the like) other than the inner tube 12-2_2.

The inner tube 12_2 described in the embodiment is configured as a single molded product having two lumens. On the other hand, in the inner tube 12-2_2 shown in Figs. 16A and 16B, a thread loop 36_2 is bonded to the outer peripheral surface of a tubular member 123_2. The thread loop 36_2 is produced by, for example, binding resin fibers such as PET or PE, and is fixed to the tubular member 123_2 by adhesion or welding. The thread loop 36_2 may be fixed to the tubular member 123_2 in a form in which the thread loop 36_2 is attached to a small molded component and the molded component is attached to the outer peripheral surface of the tubular member 123_2.

An inner space of the tubular member 123_2 forms the first guide portion 31_2, and a space between the outer peripheral surface of the tubular member 123_2 and the thread loop 36_2 forms the second guide portion 32_2. That is, the second guide portion 32_2 is provided outside the inner tube 12-2_2 (axial member). In this case, the insertion and the pulling out of the restraining strap 21_2 and the holding wire 22_2 can be easily performed.

The inner tube 12-2_2 can be easily produced as compared with the inner tube 12_2 consisting of the integral multi-lumen tube described in the embodiment, because the inner tube 12-2_2 can be produced only by bonding the thread loop 36_2 to the outer peripheral surface of the tubular member 123_2. In addition, since the thread loop 36_2 can be easily deformed and has an extremely small influence on the filling rate inside the sheath 11_2, the operability when releasing the colonic stent 2_2 from the sheath 11_2 is also ensured.

The thread loop 36_2 may be provided such that the thread loop 36_2 is bent to alternately form concave portions and convex portions in the circumferential direction, with the concave portion bonded to the outer peripheral surface of the tubular member 123_2, and the convex portion bulging radially outward with respect to the outer peripheral surface of the tubular member 123_2. In this case, a plurality of the second guide portions 32_2 can be easily formed, and the plurality of conversion means 20_2 can be individually guided.

Although the invention made by the inventors has been specifically described above based on the embodiment, the present invention is not limited to the above-described embodiment and can be changed without departing from the gist of the present invention.

For example, the structures of the inner tubes 12_2, 12-1_2, and 12-2_2 shown in the second embodiment and Modification Examples 1 and 2 are examples, the present invention is not limited to these, and changes can be freely made as appropriate. In a case where the positional deviation of the colonic stent 2_2 in the axial direction can be sufficiently suppressed by the frictional force exerted by the stent attachment portion 33_2 of the inner tube 12_2, the protruding rib 35_2 need not be provided.

In addition, for example, in the second embodiment, the first to third conversion means 20A_2 to 20C_2 having independent configurations are provided, but this is merely an example, and the present invention is not limited to this, and the restraining strap 21_2 and the holding wire 22_2 may be used in common. For example, the shape, the length, and the like of the restraining strap 21_2 or the holding wire 22_2 may be adjusted such that the colonic stent 2_2 is released in a predetermined order according to the operation amount or the operation mode. Further, the number of the conversion means is merely an example and is not limited to this, and at least one conversion means may be provided.

In addition, in the second embodiment, a case has been described in which the entire axial portion of the colonic stent 2_2 is maintained in a contracted state by the first to third conversion means 20A_2 to 20C_2, but the conversion means 20_2 need only maintain at least a portion of the colonic stent 2_2 in a contracted state. For example, the first and second conversion means 20A_2 and 20B_2 may be configured to maintain, at the time of the release from the sheath 11_2, the first stent portion 2A_2 and the second stent portion 2B_2 of the colonic stent 2_2 in a contracted state, and the third stent portion 2C_2 may be configured to be deployed with the release from the sheath 11_2.

In addition, in the second embodiment, the colonic stent 2_2 which is a straight type bare stent has been described as an example of the tubular indwelling device to be placed by the delivery system 100_2, but this is merely an example, and the present invention is not limited to this. For example, as the tubular indwelling device to be placed by the delivery system 100_2, a single-side or double-side flared type stent can be applied. Further, the tubular indwelling device may be a covered stent in which a membrane is disposed entirely or partially on the peripheral surface of the skeleton. However, in a case of the covered stent, in order to extract the conversion means 20_2 from the inside to the outside, a mesh type having pores is suitable for the membrane.

Furthermore, the present invention is not limited to the colonic stent 2_2 described in the embodiment and can be applied to a digestive tract stent that is placed in a digestive tract such as an esophagus or a colon, or a stent graft that is placed in a blood vessel. In this case, a fluid flowing through the digestive tract includes, for example, food immediately after ingestion before any digestion has occurred, a substance that has been broken down as food passes through the digestive tract, a substance that has not been digested even after passing through the digestive tract (for example, feces or the like), and the like, regardless of the state of the substance.

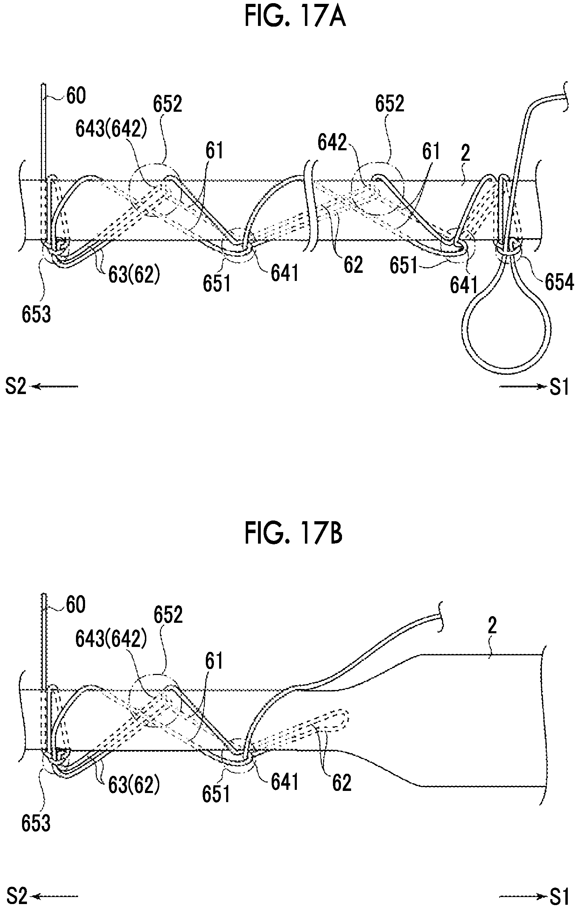

In the first embodiment, as shown in Fig. 2, the conversion means 20 is composed of the restraining strap 21 and the holding wire 22, but may be composed of only the restraining strap 21. A restraining method in a case where the conversion means 20 is composed of only the restraining strap 21 will be described with reference to Fig. 17A and the like.

Although the first to third conversion means 20A to 20C are composed of the restraining straps 21A to 21C different from each other, the restraining method is the same. Therefore, descriptions will be provided by using a "restraining strap 60" below.

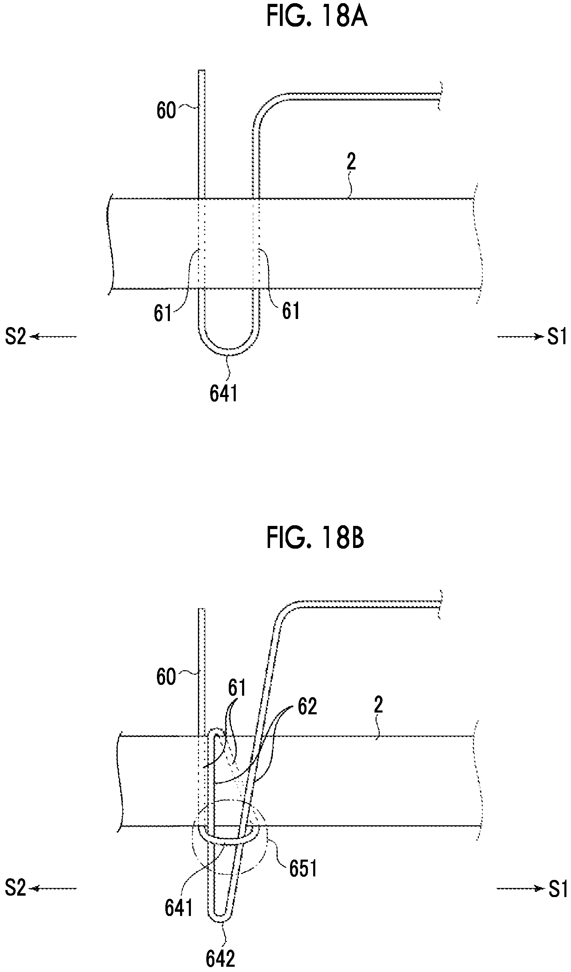

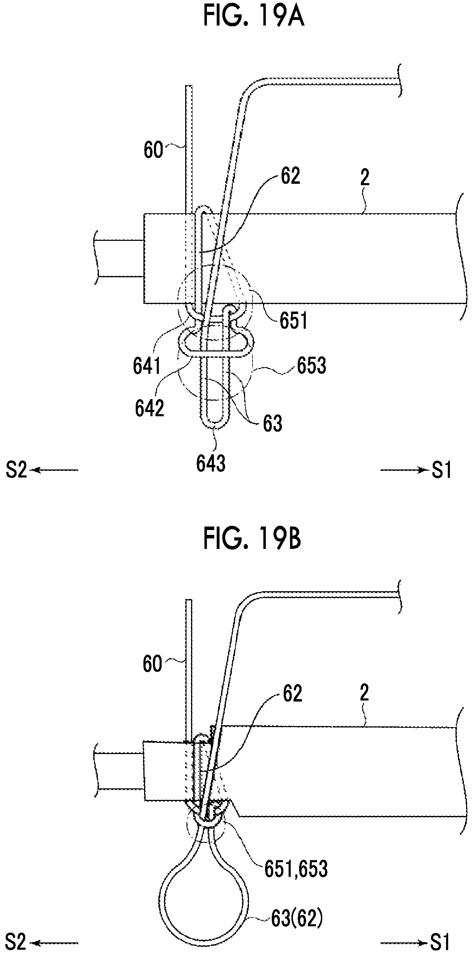

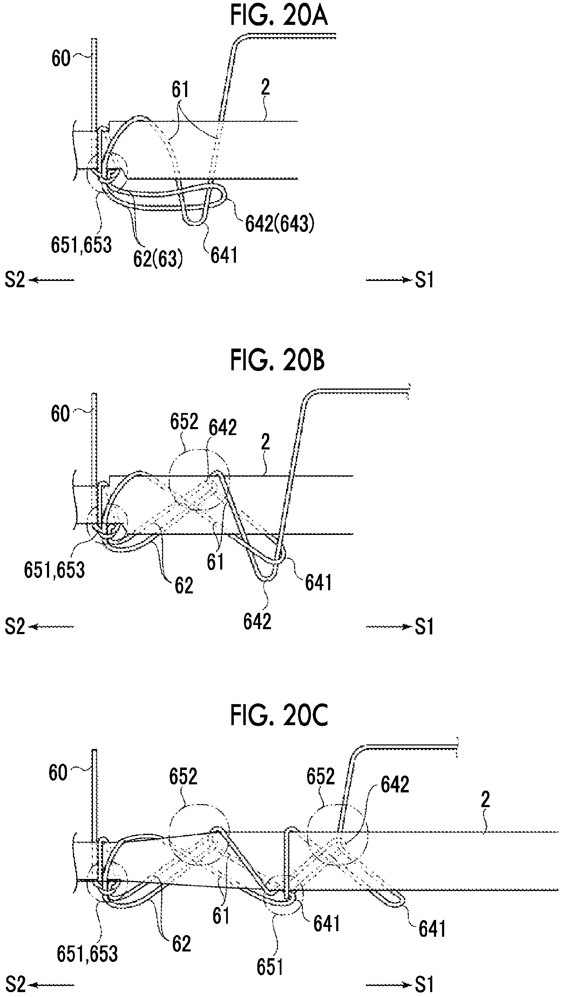

Fig. 17A is a schematic view showing a state in which the colonic stent 2 is restrained by the restraining strap 60. Fig. 17B is a schematic view showing a state in which the restraint of the colonic stent 2 by the restraining strap 60 is partially released. Figs. 18A, 18B, 19A, and 19B are views illustrating the restraint of the end part of the colonic stent 2. Figs. 20A to 20C are views illustrating the restraint of the central part of the colonic stent 2.

As shown in Fig. 17A, temporary tying portions 653 and 654 are formed by the restraining strap 60 at both end parts of the colonic stent 2. The temporary tying portions 653 and 654 have a fixing force to an extent that temporary tying portions 653 and 654 are not released in a natural state but are released when the operator pulls the restraining strap 60. Here, the fixing force in the temporary tying portions 653 and 654 is ensured by the knotting of the restraining strap 60.

In addition, at the central part of the colonic stent 2, a first knotting portion 651 and a second knotting portion 652 are alternately formed along the axial direction by the restraining strap 60. The colonic stent 2 is restrained by the restraining strap 60 and is maintained in a contracted state.

In a case where the colonic stent 2 is restrained by using a single restraining strap 60, first, the temporary tying portion 653 is formed on the distal side S2 of the colonic stent 2. Specifically, as shown in Fig. 18A, an end part of the restraining strap 60 on the distal side S2 is bent to double the restraining strap 60, and the doubled portion (a portion forming a bending portion) is wound along a peripheral surface part (on a background side of the peripheral surface part of the colonic stent 2 in Fig. 18A) of the colonic stent 2 in a first winding direction. In Fig. 18A, the first winding direction is counterclockwise when viewed from the distal side S2. Hereinafter, a portion that is wound in the first winding direction is referred to as a "first bending portion forming part 61", and a bending portion formed by the first bending portion forming part 61 is referred to as a "first bending portion 641".

Next, as shown in Fig. 18B, a portion of the restraining strap 60 extending from the first bending portion forming part 61 toward the proximal side S1 is bent to double the portion, and the doubled portion (a portion forming a bending portion) is wound along the peripheral surface part of the colonic stent 2 (in Fig. 18B, on a foreground side of the peripheral surface part of the colonic stent 2) in a second winding direction. In Fig. 18B, the second winding direction is clockwise when viewed from the distal side S2. Hereinafter, a portion that is wound in the second winding direction is referred to as a "second bending portion forming part 62", and a bending portion formed by the second bending portion forming part 62 is referred to as a "second bending portion 642".

The first knotting portion 651 is formed by passing the second bending portion 642 formed by the second bending portion forming part 62 axially adjacent to the first bending portion forming part 61 through an inner side of the first bending portion 641 formed by the first bending portion forming part 61.

Further, as shown in Fig. 19A, a portion of the restraining strap 60 extending from the second bending portion forming part 62 toward the proximal side S1 is bent to double the portion, and the doubled portion (a portion forming a bending portion) is wound along the peripheral surface part of the colonic stent 2 (in Fig. 19A, on the foreground side of the peripheral surface part of the colonic stent 2) in the second winding direction. Hereinafter, a portion that is axially adjacent to the second bending portion forming part 62 and that is wound in the second winding direction is referred to as a "temporary tying bending portion forming part 63", and a bending portion formed by the temporary tying bending portion forming part 63 is referred to as a "temporary tying bending portion 643".

The temporary tying portion 653 is formed by passing the temporary tying bending portion 643 of the temporary tying bending portion forming part 63 adjacent to the second bending portion forming part 62 through an inner side of the second bending portion 642 of the second bending portion forming part 62. By disposing the first knotting portion 651 and the temporary tying portion 653 to overlap with each other in the axial direction, the restraining strap 60 is less likely to be released as compared with a case where the first knotting portion 651 is disposed alone. By providing the temporary tying portion 653 at the end part of the colonic stent 2 on the distal side S2, it is possible to prevent the deviation of the restraining strap 60 when forming the central part of the colonic stent 2, and it is possible to smoothly proceed with restraining work.

As shown in Fig. 20A, the temporary tying bending portion forming part 63 by which the temporary tying portion 653 has been formed is extracted to the proximal side S1 as the second bending portion forming part 62 and is used to restrain the central part of the colonic stent 2. That is, the first bending portion 641 of the first bending portion forming part 61 that is axially adjacent to the second bending portion forming part 62 and that is wound in the first winding direction is passed through the inner side of the second bending portion 642 (that is the same as the temporary tying bending portion 643 when forming the temporary tying portion 653) of the second bending portion forming part 62 extracted from the temporary tying portion 653 on the distal side S2. Then, the first bending portion 641 passed through the second bending portion 642 is pulled toward the proximal side S1, so that the second bending portion forming part 62 is tied up to form the second knotting portion 652 (refer to Fig. 20B).

Further, as shown in Fig. 20B, the second bending portion 642 of the second bending portion forming part 62 that is axially adjacent to the first bending portion forming part 61 and that is wound in the second winding direction is passed through the inner side of the first bending portion 641 of the first bending portion forming part 61 extracted from the second knotting portion 652. Then, the second bending portion 642 passed through the first bending portion 641 is pulled toward the proximal side S1, so that the first bending portion forming part 61 is tied up to form the first knotting portion 651 (refer to Fig. 20C). The first knotting portion 651 and the second knotting portion 652 are disposed at radially facing positions (positions rotated by approximately 180°) when viewed from the axial direction.

In the restraining strap 60, a portion where the first knotting portion 651 and the second knotting portion 652 are connected extends obliquely to have a circumferential component and an axial component and does not include a portion that extends linearly in the axial direction. That is, since the restraining strap 60 does not have the portion that is formed to linearly extend in the axial direction, which does not function as a restraining portion for the colonic stent 2, the length of the restraining strap 60 that is wound around the peripheral surface part of the colonic stent 2 to restrain the colonic stent 2 can be shortened.

As described above, by alternately forming the first knotting portion 651 and the second knotting portion 652 in the axial direction while intersecting the first bending portion forming part 61 and the second bending portion forming part 62 of the restraining strap 60 with each other, the central part of the colonic stent 2 is restrained in order from the distal side S2 and is maintained in a contracted state.

In addition, the temporary tying portion 654 is formed at the end part of the colonic stent 2 on the proximal side S1 in the same manner as the temporary tying portion 653 provided at the end part on the distal side S2. By providing the temporary tying portion 654 at the end part of the colonic stent 2 on the proximal side S1, it is possible to prevent the restraint at the central part of the colonic stent 2 from being easily released. An end part of the restraining strap 60 on the proximal side S1 is, for example, extracted from the branch port 143a on one side of the Y connector 143 of the second operation part 14 and connected to the pulling-out operation portion 141 (refer to Fig. 1A) .

The restraining strap 60 is pulled toward the proximal side S1, so that the temporary tying portion 654 that restrains the end part of the colonic stent 2 on the proximal side S1 is released, and then the first knotting portion 651 and the second knotting portion 652 that restrain the central part of the colonic stent 2 are sequentially released from the proximal side S1, and the restraint of the colonic stent 2 is released (refer to Fig. 17B). A portion where the restraint of the colonic stent 2 is released is radially expanded. Finally, the temporary tying portion 653 that restrains the end part of the colonic stent 2 on the distal side S2 is released, and the colonic stent 2 is in a completely expanded state and is appropriately placed in the target indwelling position.

In the example shown in Fig. 17A and the like, the second knotting portion 652 is the first knotting portion that restrains the central part of the colonic stent 2, but the first knotting portion 651 may serve as the first knotting portion. For example, the temporary tying portion 653 may be formed by forming the first knotting portion 651 and the second knotting portion 652 at the end part of the colonic stent 2 on the distal side S2, then by winding the temporary tying bending portion forming part 63 adjacent to the first bending portion forming part 61, by which the second knotting portion 652 has been formed, in the same first winding direction as in the first bending portion forming part 61, and by passing the temporary tying bending portion 643 formed by the temporary tying bending portion forming part 63 through the inner side of the first bending portion 641 formed by the first bending portion forming part 61. In this case, since the temporary tying bending portion forming part 63 by which the temporary tying portion 653 has been formed is extracted to the proximal side S1 as the first bending portion forming part 61, the first knotting portion 651, which is formed by passing the second bending portion 642 formed by the second bending portion forming part 62 axially adjacent to the first bending portion forming part 61, serves as the first knotting portion that restrains the central part of the colonic stent 2.

In addition, the temporary tying portions 653 and 654 may be formed of an adhesive instead of the knotting of the restraining strap 60.

In a case where the restraining method shown in Fig. 17A and the like is applied to the catheter 1 of the first embodiment, the catheter 1 has the following features.

That is, the linear member that maintains the colonic stent 2 (tubular indwelling device) in a contracted state is configured as a single restraining strap 60, and the restraining strap 60 includes, alternately from one end side to the other end side of the restraining strap 60 along the axial direction, a plurality of the first bending portion forming parts 61 that are disposed on the peripheral surface part of the colonic stent 2 along the first winding direction and a plurality of the second bending portion forming parts 62 that are disposed on the peripheral surface part along the second winding direction opposite to the first winding direction. The first knotting portion 651 is formed by passing the second bending portion 642 formed by the second bending portion forming part 62 axially adjacent to one first bending portion forming part 61, among the plurality of first bending portion forming parts 61, through the inner side of the first bending portion 641 formed by the one first bending portion forming part 61, and the second knotting portion 652 is formed by passing the first bending portion 641 formed by the first bending portion forming part 61 axially adjacent to one second bending portion forming part 62, among the plurality of second bending portion forming parts 62, through the inner side of the second bending portion 642 formed by the one second bending portion forming part 62. A main restraining part 65 that restrains the colonic stent 2 is formed by alternately disposing the first knotting portion 651 and the second knotting portion 652 along the axial direction, and one end or the other end of the restraining strap 60 is pulled along the axial direction, so that the first knotting portion 651 and the second knotting portion 652 disposed in the main restraining part 65 are sequentially released and the restraint of the colonic stent 2 is released.

In addition, the restraining strap 60 includes the temporary tying bending portion forming part 63 that is axially adjacent to the second bending portion forming part 62 and that is disposed along the second winding direction, and the temporary tying portion 653 is formed by passing the temporary tying bending portion 643 of the temporary tying bending portion forming part 63 through the inner side of the second bending portion 642 of the second bending portion forming part 62 by which the first knotting portion 651 has been formed, in at least one end part of both end parts of the colonic stent 2 (tubular indwelling device) in the axial direction. One end or the other end of the restraining strap 60 is pulled along the axial direction, so that the temporary tying portions 653 and 654 are released.

The above-described restraining method can also be applied to the catheter 1_2 of the second embodiment.

With the above-described configuration, the first knotting portion 651 and the second knotting portion 652 are formed at radially facing positions (positions rotated by approximately 180°) when viewed from the axial direction of the colonic stent 2, so that by pulling the restraining strap 60, a side (first side) the colonic stent 2 on which the first knotting portion 651 is formed and a side (second side) on which the second knotting portion 652 is formed can be alternately deployed (expanded). Therefore, a reaction force applied to the colonic stent 2 from the stenotic part or the like alternates between the second side and the first side. Accordingly, it is possible to suppress the occurrence of positional deviation of the colonic stent 2 during the expansion with respect to the indwelling target position of the colonic stent 2 as compared with a case where the direction of the reaction force is constant.

The embodiments disclosed herein should be considered illustrative in all respects and not restrictive. The scope of the present invention is defined by the claims, rather than the above description, and is intended to include all modifications within the meaning and scope of the claims.

The disclosures of the specification, drawings, and abstract included in Japanese application No. 2022-030220 filed on February 28, 2022 and Japanese application No. 2022-050277 filed on March 25, 2022 are incorporated by reference in their entirety into the present application.

### Reference Signs List

1: catheter
2: colonic stent (tubular indwelling device)
11: sheath
12: inner tube (axial member)
20: conversion means
21: restraining strap (linear member)
22: holding wire (linear member)
30: guide rib
40: tube attachment portion (attachment portion)
50: guide portion
100: delivery system
C: colon (body lumen)

## Claims

1. A catheter for delivering a tubular indwelling device to be placed in a body lumen, the catheter comprising:
a sheath;
an elongated axial member that is configured to move inside the sheath in an axial direction;
a plurality of guide ribs that are disposed on the axial member to be spaced apart from each other in the axial direction and are configured to position the tubular indwelling device, which is in a radially contracted state inside the sheath, in the axial direction; and
a linear member that maintains axially at least a portion of the tubular indwelling device in a contracted state, with the tubular indwelling device released from the sheath,
wherein the plurality of guide ribs each include
an attachment portion that is eccentrically provided with respect to a circumscribed circle of the guide rib in a cross-section orthogonal to the axial direction and to which the axial member is attached, and
a guide portion through which the linear member is inserted, and
the guide ribs are disposed to be displaced from each other in a circumferential direction with respect to the axial member.

2. The catheter according to Claim 1,
wherein one guide rib of the plurality of guide ribs includes a plurality of the guide portions, and
another guide rib of the plurality of guide ribs has a circumferential displacement angle with respect to the one guide rib defined based on a relative positional relationship between the plurality of guide portions of the one guide rib.

3. The catheter according to Claim 2,
wherein the other guide rib includes the plurality of guide portions, and
the one guide rib and the other guide rib are disposed such that at least one of the plurality of guide portions overlaps in the axial direction and another guide portion does not overlap in the axial direction.

4. The catheter according to Claim 3,
wherein a plurality of the linear members are provided,
the guide rib has the same number of guide portions as the number of the plurality of linear members, and
the number of linear members to be inserted through the guide portions increases as the guide rib is disposed closer to a proximal side.

5. The catheter according to any one of Claims 1 to 4,
wherein the plurality of guide ribs have the same structure.

6. The catheter according to any one of Claims 1 to 5,
wherein the linear member is formed of a single restraining strap,
the restraining strap includes, alternately from one end side to the other end side of the restraining strap along the axial direction, a plurality of first bending portion forming parts that are disposed on a peripheral surface part of the tubular indwelling device along a first winding direction and a plurality of second bending portion forming parts that are disposed on the peripheral surface part along a second winding direction opposite to the first winding direction,
a first knotting portion is formed by passing a second bending portion formed by a second bending portion forming part axially adjacent to one first bending portion forming part, among the plurality of first bending portion forming parts, through an inner side of a first bending portion formed by the one first bending portion forming part,
a second knotting portion is formed by passing a first bending portion formed by a first bending portion forming part axially adjacent to one second bending portion forming part, among the plurality of second bending portion forming parts, through an inner side of a second bending portion formed by the one second bending portion forming part,
the first knotting portion and the second knotting portion are alternately disposed along the axial direction so that a central part of the tubular indwelling device is restrained, and
one end or the other end of the restraining strap is pulled along the axial direction so that the first knotting portion and the second knotting portion disposed at the central part are sequentially released, and restraint of the tubular indwelling device is released.

7. The catheter according to Claim 6,
wherein the restraining strap includes a temporary tying bending portion forming part that is axially adjacent to the second bending portion forming part and that is disposed along the second winding direction,
a temporary tying portion is formed by passing a temporary tying bending portion of the temporary tying bending portion forming part through the inner side of the second bending portion of the second bending portion forming part by which the first knotting portion has been formed, in at least one end part of both end parts of the tubular indwelling device in the axial direction, and
the one end or the other end of the restraining strap is pulled along the axial direction so that the temporary tying portion is released.

8. A delivery system comprising:
a tubular indwelling device to be placed in a body lumen; and
a catheter for delivering the tubular indwelling device into the body lumen,
wherein the catheter includes
a sheath,
an elongated axial member that is configured to move inside the sheath in an axial direction,
a plurality of guide ribs that are disposed on the axial member to be spaced apart from each other in the axial direction and are configured to position the tubular indwelling device, which is in a radially contracted state inside the sheath, in the axial direction, and
a linear member that maintains axially at least a portion of the tubular indwelling device in a contracted state, with the tubular indwelling device released from the sheath,
the plurality of guide ribs each include
an attachment portion that is eccentrically provided with respect to a circumscribed circle of the guide rib in a cross-section orthogonal to the axial direction and to which the axial member is attached, and
a guide portion through which the linear member is inserted, and
the guide ribs are disposed to be displaced from each other in a circumferential direction with respect to the axial member.

9. A catheter for delivering a tubular indwelling device to be placed in a body lumen, the catheter comprising:
a sheath;
an elongated axial member that is configured to move inside the sheath in an axial direction and includes a first guide portion through which a guide wire is inserted; and
a linear member that maintains axially at least a portion of the tubular indwelling device in a contracted state, with the tubular indwelling device released from the sheath,
wherein the axial member includes
a positioning portion that is provided on a portion of the axial member in the axial direction and that is disposed in a state in which the tubular indwelling device, which is in a radially contracted state inside the sheath, is positioned in the axial direction, and
a second guide portion through which the linear member is inserted.

10. The catheter according to Claim 9,
wherein the second guide portion includes a guide hole provided inside the axial member along the axial direction.

11. The catheter according to Claim 10,
wherein the axial member further includes an extraction portion for extracting the linear member inserted through the guide hole to an outside of the axial member.

12. The catheter according to Claim 9,
wherein the second guide portion is provided outside the axial member.

13. The catheter according to Claim 9,
wherein the positioning portion has a greater frictional force against the tubular indwelling device as compared with a frictional force of each of the other members different from the axial member.

14. The catheter according to Claim 9,
wherein the positioning portion includes a protruding portion that protrudes radially outward from an outer peripheral surface part of the axial member and that is provided to be axially arranged with a skeleton of the tubular indwelling device.

15. The catheter according to Claim 14,
wherein a plurality of the protruding portions are provided along the circumferential direction on the axial member, and
the skeleton of the tubular indwelling device is configured to be disposed between the plurality of protruding portions.

16. The catheter according to any one of Claims 9 to 15,
wherein the linear member is formed of a single restraining strap,
the restraining strap includes, alternately from one end side to the other end side of the restraining strap along the axial direction, a plurality of first bending portion forming parts that are disposed on a peripheral surface part of the tubular indwelling device along a first winding direction and a plurality of second bending portion forming parts that are disposed on the peripheral surface part along a second winding direction opposite to the first winding direction,
a first knotting portion is formed by passing a second bending portion formed by a second bending portion forming part axially adjacent to one first bending portion forming part, among the plurality of first bending portion forming parts, through an inner side of a first bending portion formed by the one first bending portion forming part,
a second knotting portion is formed by passing a first bending portion formed by a first bending portion forming part axially adjacent to one second bending portion forming part, among the plurality of second bending portion forming parts, through an inner side of a second bending portion formed by the one second bending portion forming part,
the first knotting portion and the second knotting portion are alternately disposed along the axial direction so that a central part of the tubular indwelling device is restrained, and
one end or the other end of the restraining strap is pulled along the axial direction so that the first knotting portion and the second knotting portion disposed at the central part are sequentially released, and restraint of the tubular indwelling device is released.

17. The catheter according to Claim 16,
wherein the restraining strap includes a temporary tying bending portion forming part that is axially adjacent to the second bending portion forming part and that is disposed along the second winding direction,
a temporary tying portion is formed by passing a temporary tying bending portion of the temporary tying bending portion forming part through the inner side of the second bending portion of the second bending portion forming part by which the first knotting portion has been formed, in at least one end part of both end parts of the tubular indwelling device in the axial direction, and
the one end or the other end of the restraining strap is pulled along the axial direction so that the temporary tying portion is released.

18. A delivery system comprising:
a tubular indwelling device to be placed in a body lumen; and
a catheter for delivering the tubular indwelling device,
wherein the catheter includes
a sheath,
an elongated axial member that is configured to move inside the sheath in an axial direction and includes a first guide portion through which a guide wire is inserted, and
a linear member that maintains axially at least a portion of the tubular indwelling device in a contracted state, with the tubular indwelling device released from the sheath, and
the axial member includes
a positioning portion that is provided on a portion of the axial member in the axial direction and that is disposed in a state in which the tubular indwelling device, which is in a radially contracted state inside the sheath, is positioned in the axial direction, and
a second guide portion through which the linear member is inserted.
